(19) Europäisches Patentamt
European Patent Office
Office européen des brevets

(11) **EP 1 736 106 B1**

(12) **EUROPEAN PATENT SPECIFICATION**

(45) Date of publication and mention
of the grant of the patent:
**15.09.2010 Bulletin 2010/37**

(51) Int Cl.:
*A61B 17/22* (2006.01)

(21) Application number: **06115797.0**

(22) Date of filing: **21.06.2006**

(54) **Wire for removing an intravascular foreign body and medical instrument**

Draht zur Entfernung intravaskulärer Fremdkörper und medizinisches Instrument

Fil vasculaire servant à enlever un corps étranger et un instrument médical

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 JP 2005183984**
**26.09.2005 JP 2005278722**

(43) Date of publication of application:
**27.12.2006 Bulletin 2006/52**

(73) Proprietor: **TERUMO KABUSHIKI KAISHA**
**Tokyo 151-0072 (JP)**

(72) Inventors:
• **Nakao, Koji**
**Terumo Kabushiki Kaisha**
**Tokyo**
**Tokyo 151-0072 (JP)**
• **Obitsu, Hideshi**
**Terumo Kabushiki Kaisha**
**Kanagawa**
**Kanagawa 259-0151 (JP)**

• **Kanamaru, Takeshi**
**Terumo Kabushiki Kaisha**
**Kanagawa**
**Kanagawa 259-0151 (JP)**

(74) Representative: **TBK-Patent**
**Bavariaring 4-6**
**80336 München (DE)**

(56) References cited:
**EP-A- 1 632 188        WO-A-2005/107618**
**JP-A- 62 268 545**

• **PATENT ABSTRACTS OF JAPAN vol. 2003, no. 12, 5 December 2003 (2003-12-05) & JP 2004 016668 A (TERUMO CORP; MARUHO HATSUJO KOGYO KK), 22 January 2004 (2004-01-22)**
• **PATENT ABSTRACTS OF JAPAN vol. 2002, no. 02, 2 April 2002 (2002-04-02) & JP 2001 276081 A (OLYMPUS OPTICAL CO LTD), 9 October 2001 (2001-10-09)**
• **PATENT ABSTRACTS OF JAPAN vol. 2003, no. 12, 5 December 2003 (2003-12-05) & JP 2005 131041 A (TERUMO CORP), 26 May 2005 (2005-05-26)**

## Description

The present invention relates to a wire for removing an intravascular foreign body such as embolus in a vessel according to the preamble of claim 1, the features of which are known from document JP 2004 016668 A, and to a medical instrument.

The vital statistics of population published by the Ministry of Health, Labor, and Welfare indicates that cancer dominates the cause of Japanese death, while heart disease and cerebral apoplexy represent the second and third leading causes of Japanese death. The increasing deaths and sequelae due to cerebral apoplexy urgently demand to establish a therapeutic method for cerebral apoplexy.

A recent development in therapy of cerebral apoplexy is thrombolysis involving use of a thrombolytic agent to cure brain infarction in its acute phase. This therapy is effective, but its effectiveness is limited. That is, the thrombolytic agent takes a long time for thrombolysis or produces smaller thrombi that scatter to form new emboli. In addition, through experiences of doctors, it has been found that some emboli are insoluble by treatment with a thrombolytic agent.

It has been proved in the U.S. and Europe that the probability to save lives and reduce sequelae would be high if the blood flow is resumed within 3 hours after the onset of cerebral apoplexy. Thus, there is a strong interest in developing a new medical instrument that can be inserted into a cerebral vessel to remove the thrombus directly. An example of such a medical instrument is a wire for removing an intravascular foreign body which is disclosed in JP 2004 016 668 A.

The wire for removing an intravascular foreign body is composed of a wire body, two branch wire parts which branch out from the wire body, and a plurality of filament parts bridged between the branch wire parts. The branch wire parts and the filament parts form a space in which an intravascular foreign body is captured.

The disadvantage of this disclosed wire for removing an intravascular foreign body is that, depending on the size of the foreign body, the wire is sometimes not able to capture an intravascular foreign body.

For example, if the intravascular foreign body is smaller than the space in which it is to be captured, the intravascular foreign body might slip through the gap between the filament parts. In such a case, it would be necessary to exchange the wire for removing an intravascular foreign body with a different that matches the size of the foreign body to be captured. This is troublesome.

Document JP 62 268 545 A discloses a wire for removing an intravascular foreign body having a plurality of filaments at a distal end. The filaments are arranged about a center axis extending from the wire. At its one end, each of the filaments is connected with the wire, while on its other end the filaments are connected with each other. Thus, a basket like space for capturing the foreign body is formed.

To improve the capturing ability of the wire, apart from being formed more or less straight, a portion of each of the filaments can be formed in a coiled shape.

It is the object of the present invention to provide a wire and a medical instrument surely capable to capture an intravascular foreign body and to remove the intravascular foreign body such as an embolus in a blood vessel.

The object of the present invention is achieved by a wire according to claim 1 or by a medical instrument according to claim 18. The wire of the invention can be as follows:

(1) A wire for removing an intravascular foreign body, comprises:

a long or elongated wire body with flexibility; and
a capturing portion including a foreign body capturing space for capturing a foreign body in a blood vessel therein the foreign body capturing space, the capturing portion being provided on a distal end of the wire body,
wherein the capturing portion includes:

at least two branch wires branching from the distal end of the wire body; and
a plurality of filaments bridged between the two branch wires, and
at least one of the filaments includes a plurality of curved projections being second filaments bent in a direction deviated from a plane defined by the first filaments in a natural state each of said second filaments forming a loop.

(2) The wire for removing an intravascular foreign body according to (1), wherein each of the plurality of curved projections includes a first curved projection bent in a direction deviated in a first direction from the extension line of the central axis of the proximal end of the filament in a natural state, and a second curved projection bent in a direction deviated from the extension line of the central axis of the proximal end of the filament oppositely to the first direction. With the wire according to (1) the second filaments can be branching from midway of the at least one of the first filaments.

(4) The wire for removing an intravascular foreign body according to (3), wherein a sum of $\alpha$ and $\beta$ satisfies a following expression in a natural state:

$$45° \leq \alpha+\beta \leq 90°$$

where $\alpha$ is an angle made by an extension line of a central axis of each of the branch wires and an extension line of a central axis of a proximal end of each of the first filaments, and β is an angle made by the extension line of the central axis of the proximal end of each of the first filaments and an extension line of a central axis of a proximal end of each of the second filaments.

(5) The wire for removing an intravascular foreign body according to (3), wherein positions of branch points of the plurality of second filaments coincide with each other substantially.

(6) The wire for removing an intravascular foreign body according to (3), wherein positions of branch points of the plurality of second filaments are different to each other.

(7) The wire for removing an intravascular foreign body according to any one of (3) to (6), wherein the capturing portion includes a third filament on at least one of the second filaments, the third filament branching from midway of the at least one of the second filament.

(8) The wire for removing an intravascular foreign body according to (7), wherein a plurality of the third filaments are provided on the at least one of the second filaments.

(9) The wire for removing an intravascular foreign body, according to (3), wherein

at least one of the filaments includes a bent and deformed portion having a plurality of bent or steeply curved inflection points, and a coupling portion located between two of the inflection points and disposed to enter a space between the filaments adjacent to each other.

(10) The wire for removing an intravascular foreign body according to (9), wherein the bent and deformed portion has a plurality of the coupling portions, and the coupling portions adjacent to each other are extended in directions different from each other.

(11) The wire for removing an intravascular foreign body according to (10), wherein the bent and deformed portion forms a wave shape when viewed from a distal end side of the wire body in a longitudinal direction.

(12) The wire for removing an intravascular foreign body according to (10) or (11), wherein each of the plurality of filaments has the bent and deformed portion forming a wave shape, and in the bent and deformed portion adjacent to each other, each of the inflection points of one of the filaments enters a space between the two coupling portions of the other.

(13) The wire for removing an intravascular foreign body according to (11) or (12), wherein, in the bent and deformed portion forming the wave shape, amplitude of the bent and deformed portion on the distal end side is larger than amplitude of the bent and deformed portion on a proximal end side.

(14) The wire for removing an intravascular foreign body according to any one of (9) to (13), wherein the bent and deformed portion is composed of a linear body having portions different in thickness.

(15) The wire for removing an intravascular foreign body according to any one of (9) to (14), wherein each of the plurality of filaments has the bent and deformed portion, and the bent and deformed portion partially contact or intersect each other.

(16) The wire for removing an intravascular foreign body according to any one of (9) to (15), wherein the bent and deformed portion is superior in pliability than the branch wires.

(17) The wire for removing an intravascular foreign body according to any one of (9) to (16), wherein the plurality of filaments are capable of approaching and being spaced apart from one another.

The medical instrument of the invention can be as follows :

(18) A medical instrument, comprising:

the wire for removing an intravascular foreign body according to any wire of (1) to (17); and a catheter provided with a lumen capable of housing the wire for removing an intravascular foreign body in the lumen.

(19) The medical instrument according to (18), wherein distal ends of the branch wires are spaced apart from each other when the capturing portion is allowed to project from a distal end opening of the lumen; and the distal ends of the branch wires come close to each other by being regulated by an inner wall surface which defines the lumen when the capturing portion is housed in the lumen.

In accordance with the wire for removing an intravascular foreign body according to the present invention, the wire for removing an intravascular foreign body comprises:

a long or elongated wire body with flexibility; and a capturing portion including a foreign body capturing space for capturing a foreign body in a blood vessel therein the foreign body capturing space, the capturing portion being provided on a distal end of the wire body, wherein the capturing portion includes:

at least two branch wires branching from the distal end of the wire body; and a plurality of filaments bridged between the two branch wires, and at least one of the filaments includes a plurality of curved projections bent in a direction deviated from an extension line of a central axis of a proximal end of the filament in a natural state. Thus making it possible to surely capture and remove the foreign body in the blood vessel.

In accordance with a first aspect of the wire for removing an intravascular foreign body according to the present invention, the wire for removing an intravascular foreign body comprises:

a long or elongated wire body with flexibility; and a capturing portion including a foreign body capturing space for capturing a foreign body in a blood vessel in the foreign body capturing space, the capturing portion being provided on a distal end of the wire body,

wherein the capturing portion includes:

at least two branch wires branching from the distal end of the wire body; a plurality of first filaments bridged between the two branch wires; and a plurality of second filaments on at least one of the first filaments, the second filaments branching from midway of the at least one of the first filaments. Thus, a contact area of the capturing portion with the foreign body within the blood vessel in the capturing portion concerned becomes large. Thus, the foreign body in the blood vessel can be captured and removed more surely.

In the first aspect of the wire for removing an intravascular foreign body according to the present invention, when the third filament is provided, the contact area with the foreign body in the blood vessel within the capturing portion becomes larger, and therefore, the foreign body in the blood vessel can be captured and removed more surely.

In accordance with a second aspect of the wire for removing an intravascular foreign body according to the present invention, the wire for removing an intravascular foreign body comprises:

a long or elongated wire body with flexibility; and a capturing portion including a foreign body capturing space for capturing a foreign body in a blood vessel in the foreign body capturing space, the capturing portion being provided on a distal end of the wire body,

wherein the capturing portion includes:

at least two branch wires branching from the wire body; and a plurality of filaments bridged between the two branch wires, and at least one of the filaments includes a bent and deformed portion having a plurality of bent or steeply curved inflection points, and a coupling portion located between two of the inflection points and disposed to enter a space between the filaments adjacent to each other. Thus making it possible to capture and remove the foreign body in the blood vessel more surely.

In the accompanying drawings:

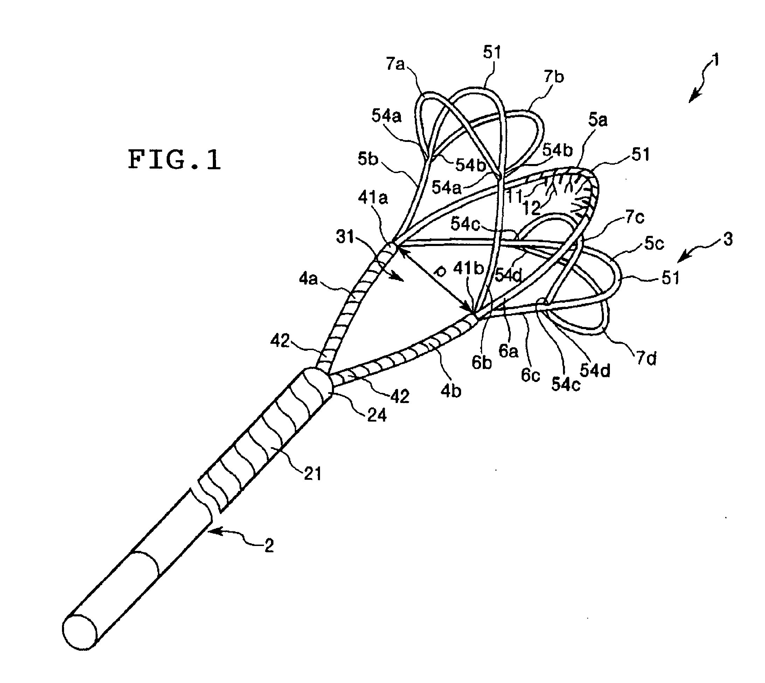

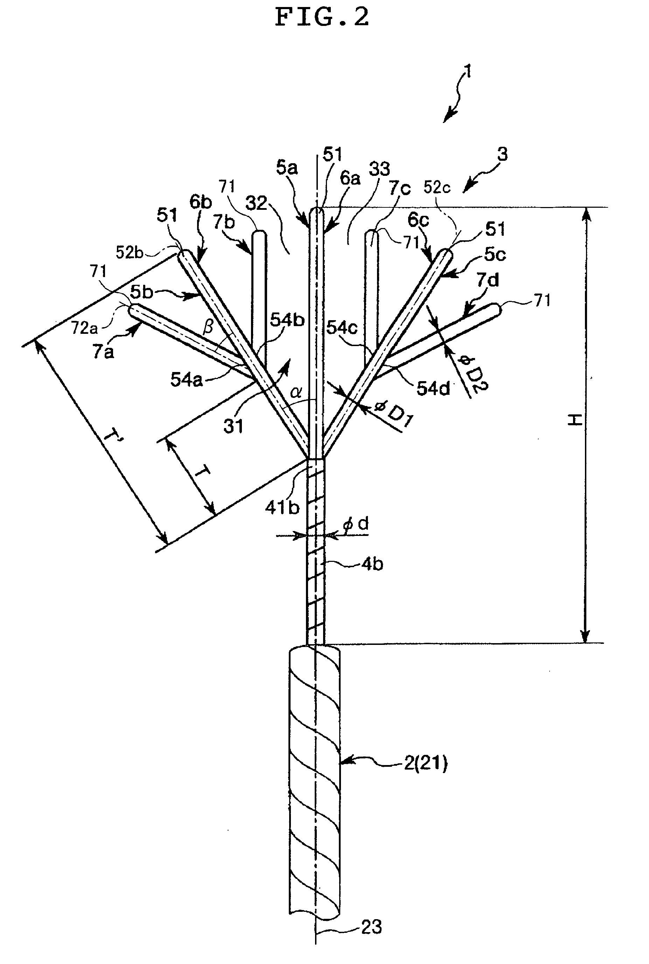

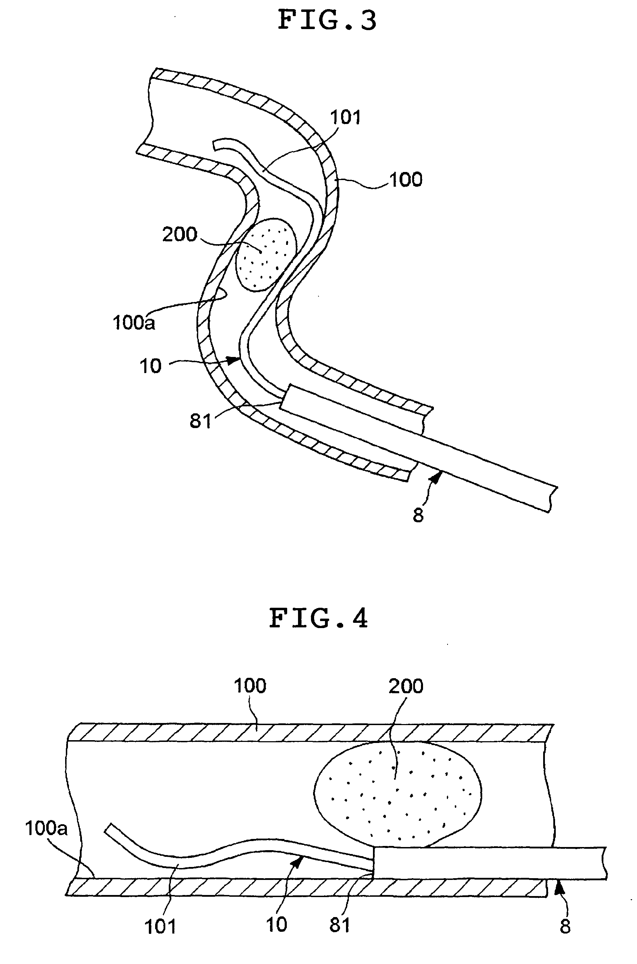

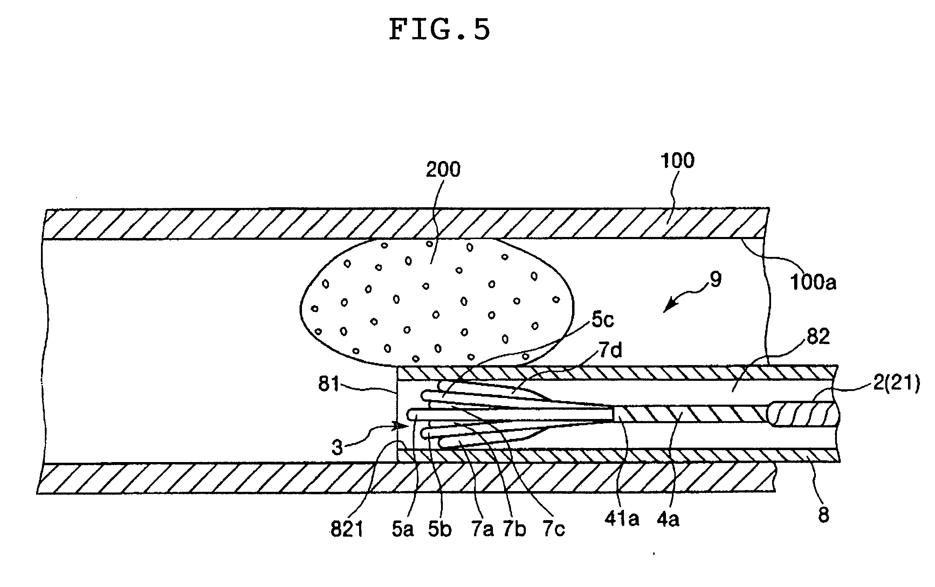

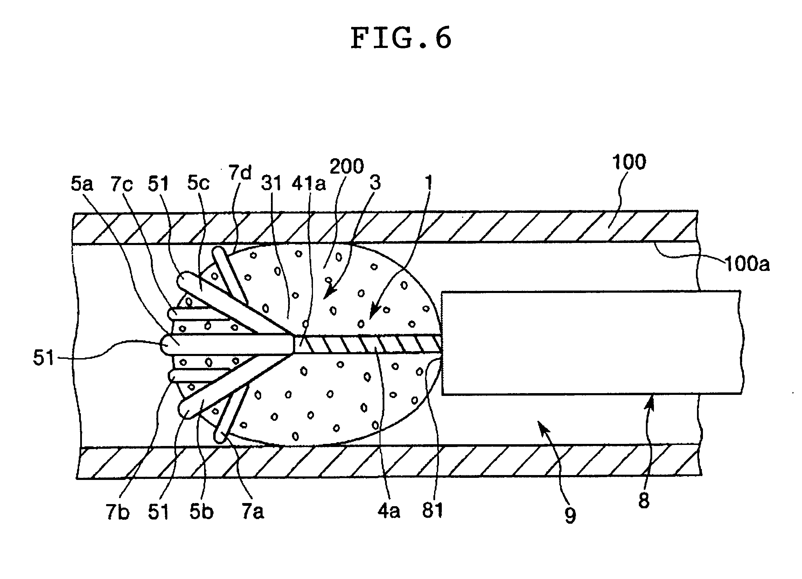

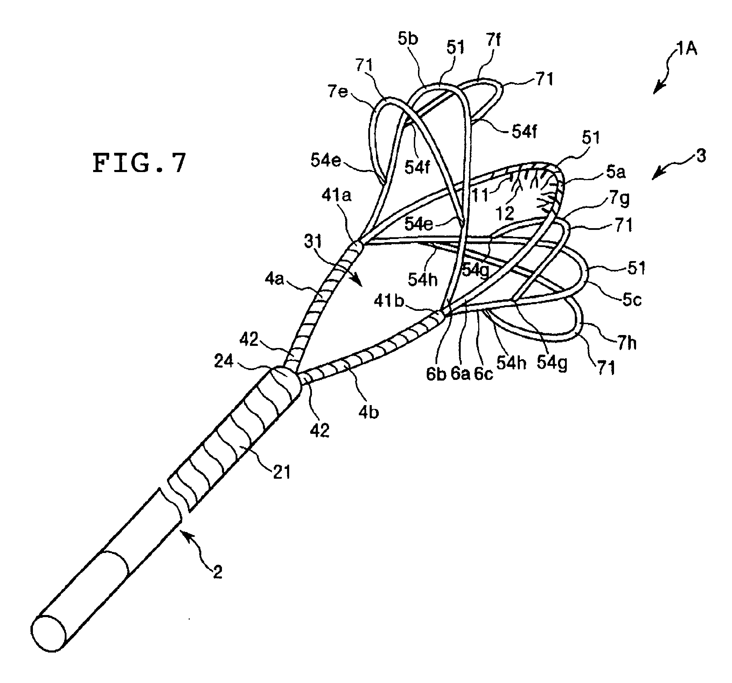

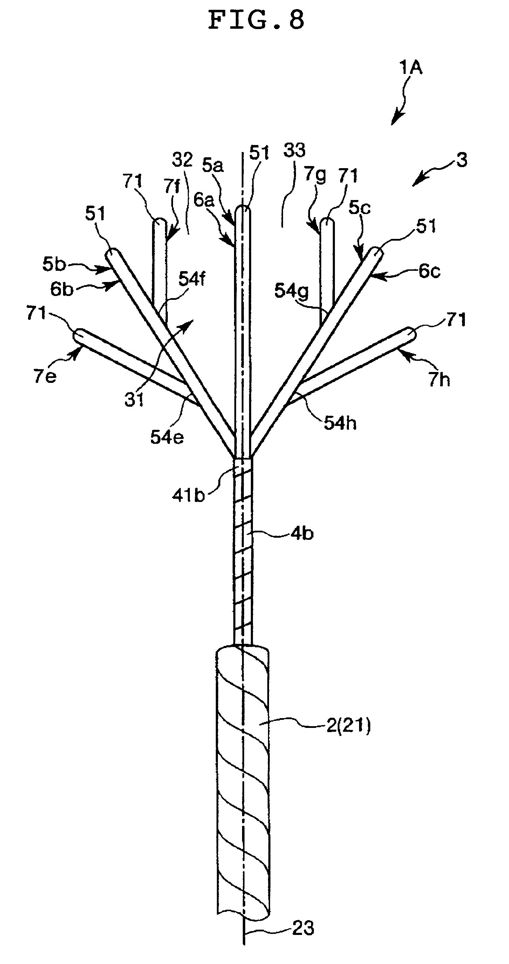

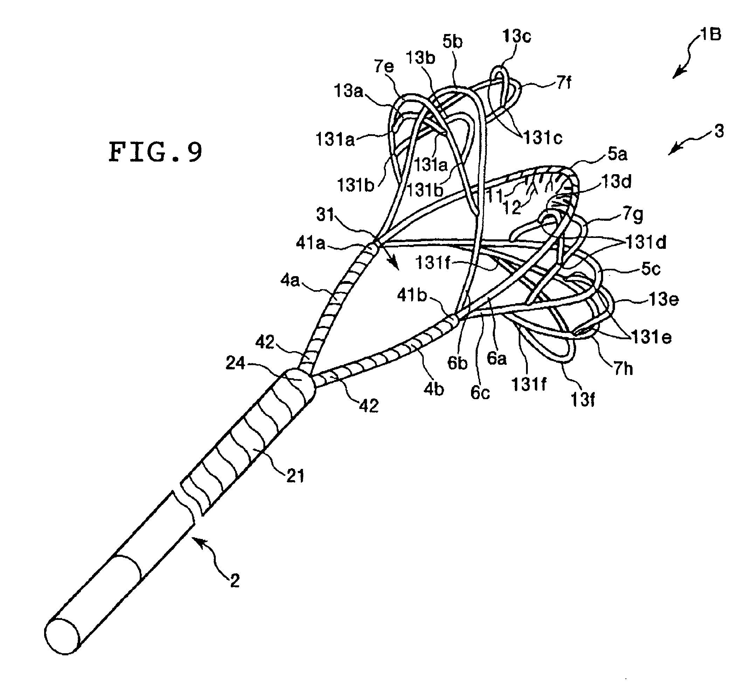

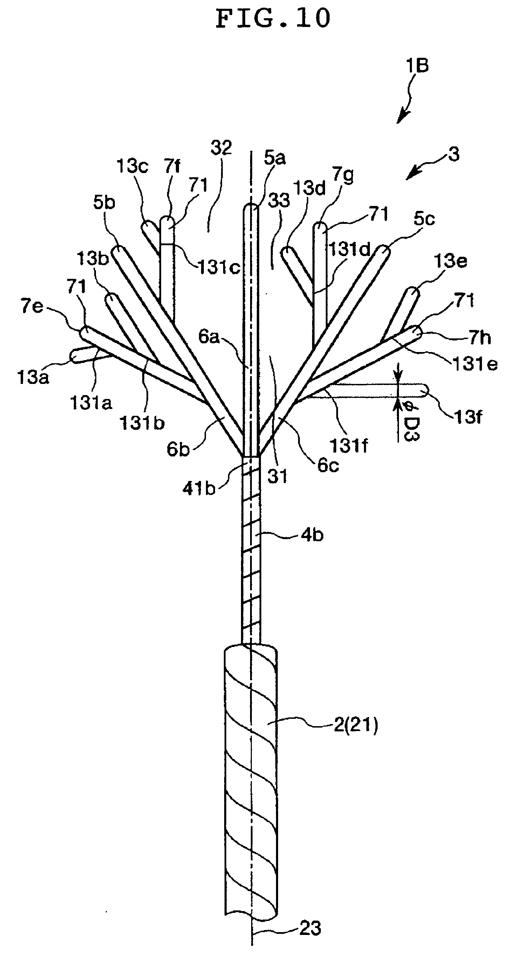

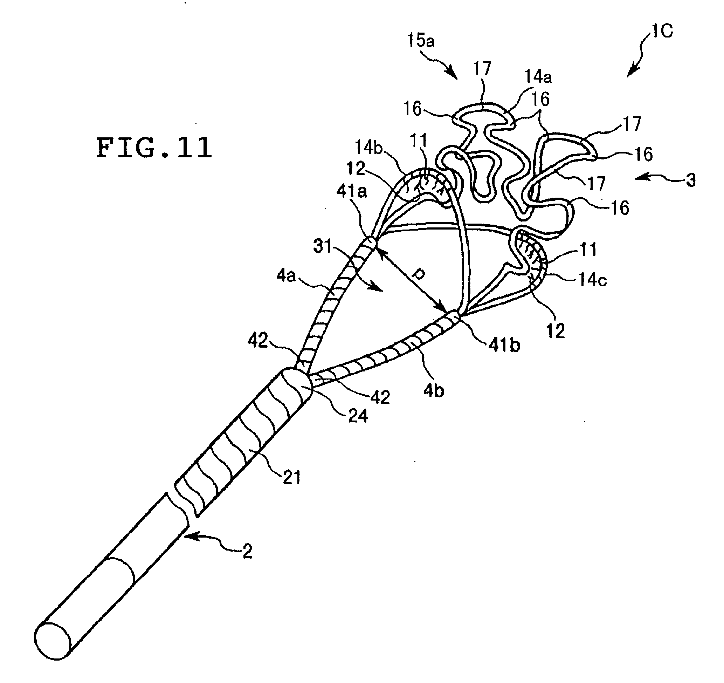

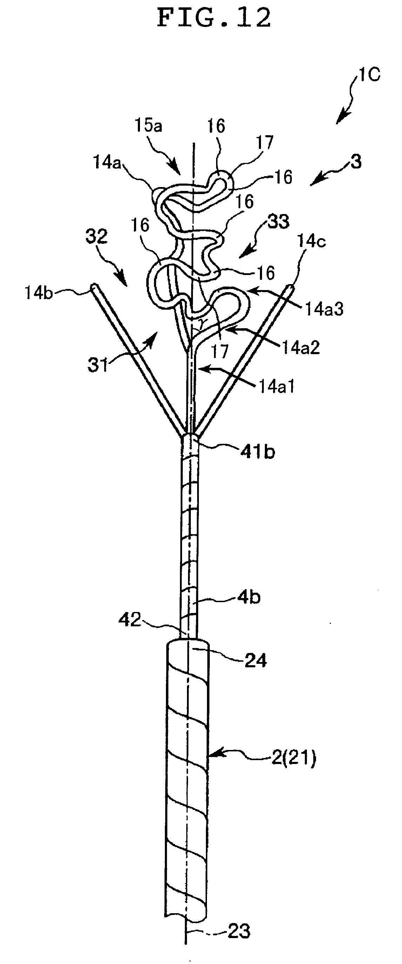

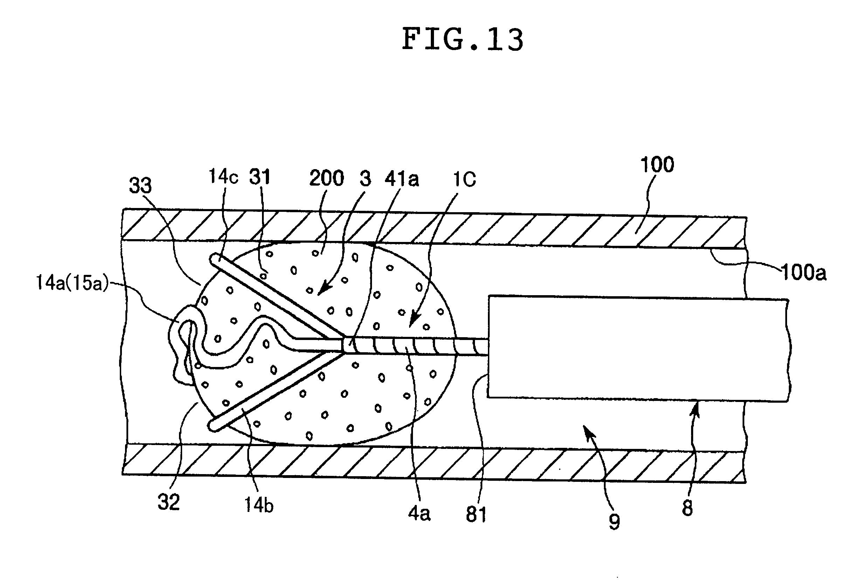

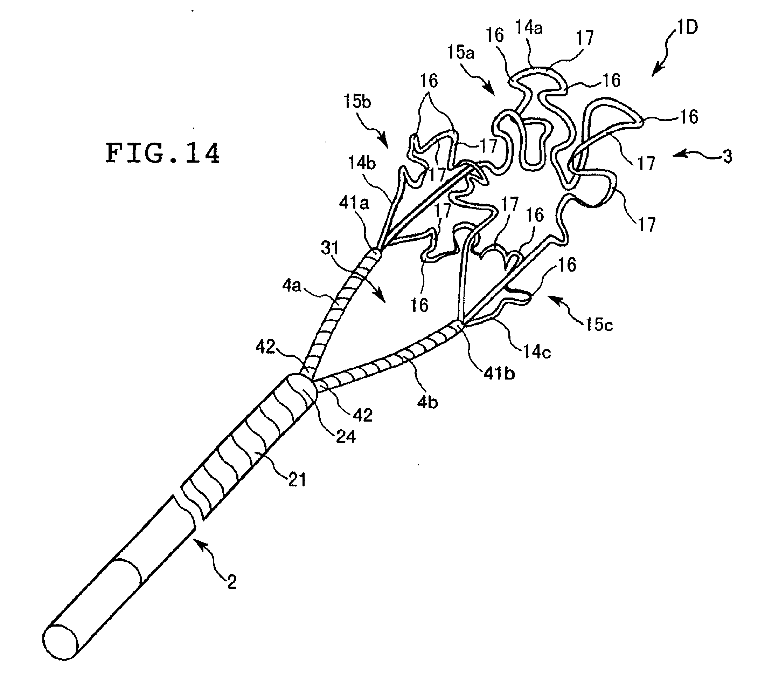

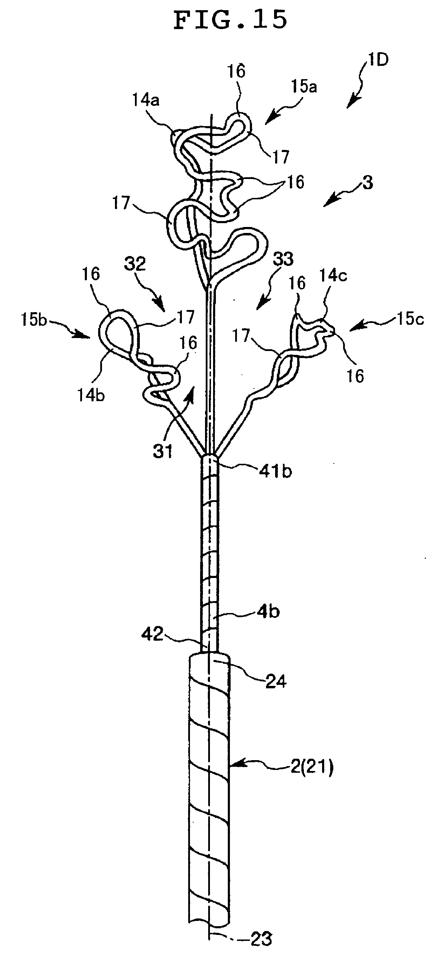

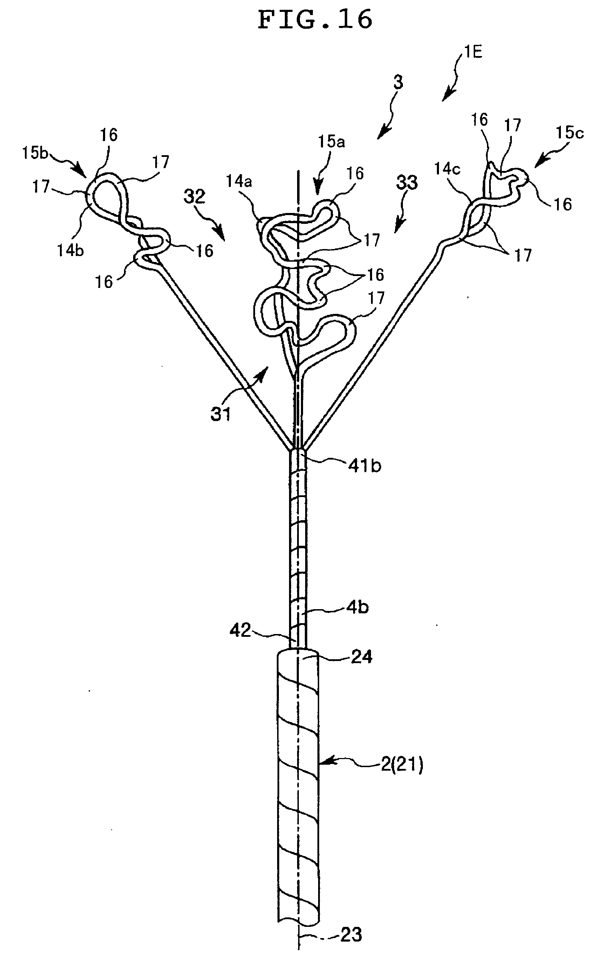

FIG. 1 is a perspective view of a wire for removing an intravascular foreign body according to a first embodiment (natural state) of the present invention; FIG. 2 is a side view of the wire for removing an intravascular foreign body shown in FIG. 1; FIG. 3 is a general illustration of a manner of using the wire for removing an intravascular foreign body shown in FIG. 1; FIG. 4 is a general illustration of a manner of using the wire for removing an intravascular foreign body which is shown in FIG. 1; FIG. 5 is a general illustration of a manner of using the wire for removing an intravascular foreign body which is shown in FIG. 1; FIG. 6 is a general illustration of a manner of using the wire for removing an intravascular foreign body which is shown in FIG. 1; FIG. 7 is a perspective view of a wire for removing an intravascular foreign body according to a second embodiment (natural state) of the present invention; FIG. 8 is a side view of the wire for removing an intravascular foreign body shown in FIG. 7; FIG. 9 is a perspective view of a wire for removing an intravascular foreign body according to a third embodiment (natural state) of the present invention; FIG. 10 is a side view of the wire for removing an intravascular foreign body shown in FIG. 9; FIG. 11 is a perspective view of a wire for removing an intravascular foreign body according to a first (natural state) comparative example for better understanding the present invention; FIG. 12 is a side view of the wire for removing an intravascular foreign body shown in FIG. 11; FIG. 13 is a view showing a state where an embolus in a blood vessel is captured by using the wire for removing an intravascular foreign body, which is shown in FIG. 12; FIG. 14 is a perspective view of a wire for removing an intravascular foreign body according to a second (natural state) comparative example for better understanding the present invention; FIG. 15 is a side view of the wire for removing an intravascular foreign body shown in FIG. 14; FIG. 16 is a side view of a wire for removing an intravascular foreign body according to a third (natural state) comparative example for better understanding the present invention;
FIG. 17 is a perspective view of a wire for removing an intravascular foreign body according to a fourth (natural state) comparative example for better understanding the present invention;
FIG. 18 is a side view of the wire for removing an intravascular foreign body shown in FIG. 17;
FIG. 19 is a view (top view) of the wire for removing an intravascular foreign body, which is shown in FIG. 17, when viewed from a distal end side;
FIGS. 20A to 20C are views (top views) showing a state where the capturing portion of the wire for removing an intravascular foreign body, which is shown in FIG. 17, captures the embolus;
FIG. 21 is a perspective view of a wire for removing an intravascular foreign body according to an fifth (natural state) comparative example for better understanding the present invention;
FIG. 22 is a side view of a wire for removing an intravascular foreign body according to a sixth (natural state) comparative example for better understanding the present invention;
FIG. 23 is a view (top view) of the wire for removing an intravascular foreign body, which is shown in FIG. 22, when viewed from a distal end side;
FIG. 24 is a perspective view of a wire for removing an intravascular foreign body according to a seventh (natural state) comparative example for better understanding the present invention; and
FIG. 25 is a side view of the wire for removing an intravascular foreign body shown in FIG. 24.

Preferred embodiments of the wire for removing an intravascular foreign body and medical instrument of the present invention will now be described in detail with reference to the drawings.
The wire for removing an intravascular foreign body according to the present invention is a wire for removing an intravascular foreign body, including a long or elongated wire body with flexibility, and a capturing portion having a foreign body capturing space for capturing the foreign body in a blood vessel therein, the capturing portion being provided on a distal end of the wire body, wherein the capturing portion includes at least two branch wires branching from the distal end of the wire body, and a plurality of filaments each of which is bridged between the two branch wires, and at least one of the filaments includes a plurality of curved projections bent in directions deviated from an extension line of a central axis of a proximal end thereof in a natural state. Here, the "natural state" refers to a state where an external force is not applied to the branch wires.
In the wire for removing an intravascular foreign body according to the present invention, it is preferable that the plurality of curved projections, in a natural state, have first curved projections bent in the direction (first direction) deviated from the extension line of the central axis, and second curved projections bent in a direction (second direction) deviated therefrom oppositely to the first direction.
A description will be made of a first aspect of the wire for removing an intravascular foreign body according to the present invention.
The first aspect of the wire for removing an intravascular foreign body according to the present invention is characterized in that the wire includes a plurality of second filaments branching from midway of the plurality of filaments (first filaments) each of which is bridged between the two branch wires.

<First Embodiment>

FIG. 1 is a perspective view of a first embodiment (natural state) of the wire for removing an intravascular foreign body, FIG. 2 is a side view of the wire for removing an intravascular foreign body shown in FIG. 1, and
FIGS. 3 to 6 are general or somewhat schematic illustrations showing a manner of use associated with the wire for removing an intravascular foreign body shown in FIG. 1.
The terms "proximal end" ("base end" or "rear end") and "distal end" ("tip end" or "forward end") in the following description are defined as follows. In FIGS. 1 and 2 (and similarly in FIGS. 7 to 12, and 14 to 25), the upper side is the "distal end side" and the lower side is the "proximal end side", while in FIGS. 3 to 6 (and similarly in FIG. 13), the right side is the "proximal end side" and the right side is the "distal end side".
Referring to FIG. 1, a wire 1 for removing an intravascular foreign body is adapted to capture and remove a foreign body, such as a thrombus and/or a clot, which can cause an embolism in a blood vessel 100. (Hereinafter, referred to as "embolus 200").
The wire 1 for removing an intravascular foreign body comprises a long or elongated wire body 2 and a capturing portion 3 attached to the distal end of the wire body 2.
Hereinafter, the composition of each part will be described.
Referring to FIG. 1, the wire body 2 has adequate rigidity and resilience (flexibility) over its entire length.
The structure of the wire body 2 is not particularly limited. For example, the wire body 2 may be one formed of a single wire, one formed by bundling a plurality of the wires, a hollow one, one with a multi-layer structure, one including a core member and a coil wound around an outer circumference thereof, one formed by combining these, or the like.
The construction materials of the wire body 2 are not particularly limited and may include metallic and plastic materials, which may be used alone or in combination.
The length of the wire body 2 may vary depending on cases such as the position and size of the blood vessel 100 to which it is applied. A preferred length ranges from 500 to 4000 mm, more preferably 1500 to 2200 mm.

The wire body 2 may also vary in outside diameter (thickness) depending on the cases such as the position and size of the blood vessel 100 to which it is applied. A preferred outside diameter is 0,1 to 2,0 mm on average, more preferably 0,25 to 0,9 mm.

The wire body 2 should preferably be composed of a first part which is comparatively hard and is located at the proximal end, a third part which is comparatively soft and is located at the distal end, and a second part which is variable in flexibility and is located at the intermediate position between the first part and the third part. In other words, the wire body 2 should preferably be formed in such a way that it gradually decreases in rigidity (flexural and torsional rigidity) from its proximal end to its distal end, that is, along a longitudinal direction. The gradually changing rigidity permits the manual manipulation to be surely transmitted to a distal end 24 of the wire body 2. With such properties making the distal end 24 relatively flexible, the wire body 2 is able to proceed and bend in the blood vessel 100 without damaging the blood vessel 100. Such properties permit the wire body 2 to transmit its twisting motion and its pushing motion while inhibiting or preventing kinking (or flexing). This contributes to higher safety.

The wire body 2 may have a coating layer on its outer surface to reduce friction resistance with the inside of a catheter 8 (described later). The coating layer permits relatively smooth insertion into and removal from the catheter. The coating layer may be formed from a fluorocarbon resin such as polytetrafluoroethylene (or Teflon(R)) or a hydrophilic polymer which becomes lubricious in a wet condition.

The capturing portion 3 includes a foreign body capturing space 31 which captures the embolus 200 therein.

As shown in FIG. 1, the capturing portion 3 includes two branch wires 4a and 4b branching from the distal end of the wire body 2, a plurality (three in this embodiment) of first filaments 5a, 5b, and 5c bridged between the branch wire 4a and the branch wire 4b, a plurality (two in this embodiment) of second filaments 7a and 7b branching from midway (midway points) of the first filament 5b, and a plurality (two in this embodiment) of second filaments 7c and 7d branching from midway (midway points) of the first filament 5c.

As shown in FIG. 1, in this embodiment, the three loop wires 6a, 6b, and 6c extending from the distal end of the wire body 2 are composed of the branch wires 4a, 4b and the first filaments 5a, 5b, 5c. Each of the loop wires 6a, 6b, and 6c extends forwardly from the distal end of the wire body 2, turns rearwards (toward a proximal end side) while forming a loop, and returns to the distal end of the wire body 2.

As shown in FIG. 1, the branch wire 4a is composed of a stranded wire formed by stranding and integrally collecting portions of the loop wires 6a, 6b, and 6c on one proximal end side.

Further, in a substantially similar way to the branch wire 4a, the branch wire 4b is composed of a stranded wire formed by stranding and integrally collecting portions of the loop wires 6a, 6b, and 6c on the other proximal end side.

The first filaments 5a, 5b, and 5c are composed so that portions of the loop wires 6a, 6b, and 6c on a distal end side can be spaced apart from one other. To be more specific, as shown in FIG. 2, the loop wires 6b and 6c are individually bent (or curved) from midway thereof toward the outsides (left and right sides in FIG. 2), and distal ends thereof distal to such bent portions individually form the first filaments 5b and 5c.

Note that, though the loop wires 6b and 6c are bent outward from the distal ends of the branch wires 4a and 4b in the illustrated construction, the present invention is not limited to this. Each of the loop wires 6b and 6c may be bent at one or a plurality of spots (few spots) in the midway between the distal ends of the branch wires 4a and 4b and an apex 51 of each of the first filaments 5b and 5c, which is to be described later. Alternatively, each of the loop wires 6b and 6c may be smoothly (gradually) curved outward from the distal ends of the branch wires 4a and 4b toward the apex 51 of each of the first filaments 5b and 5c.

As described above, the second filaments 7a and 7b are provided on the first filament 5b. Further, the second filaments 7c and 7d are provided on the first filament 5c.

A description will be made below of a construction made of the branch wires 4a and 4b, the first filaments 5a, 5b, and 5c, and the second filaments.7a, 7b, 7c and 7d.

As shown in FIG. 1, the branch wires 4a and 4b individually have linear shapes, and proximal ends 42 thereof are fixed (fixedly attached) to the distal end 24 of the wire body 2. A method of fixing the proximal ends 42 to the distal end 24 is not particularly limited; however, for example, the proximal ends 42 of the branch wires 4a and 4b can be individually fixed to the distal end 24 of the wire body 2 by performing braiding (winding), brazing, welding, adhesion using an adhesive, and so on.

In this embodiment, on the distal end 24 of the wire body 2, there is provided a coil 21 which covers fixed portions (brazed portions) of the branch wires 4a and 4b to the wire body 2. An outer surface of the coil 21 is smoothened, and higher safety is thus obtained. It is preferable that the coil 21 be formed by winding, for example, a platinum wire.

The branch wires 4a and 4b are individually composed so as to be elastically displaced (deformed), and have pliability.

As shown in FIG. 1, between a distal end 41a of the branch wire 4a and a distal end 41b of the branch wire 4b, the three filaments 5a, 5b, and 5c which form linear shapes are provided so as to be bridged therebetween. These first filaments 5a, 5b, and 5c individually form arch shapes (curved shapes) in which center portions are curved so as to bulge forward or toward the distal end side. The distal end 41a of the branch wire 4a and the distal end 41b of the branch wire 4b are connected to each other through the arch-like apices 51 (the distal end side portions of filaments 5a, 5b, and 5c) while spacing the arch-like apices 51 apart from one another (refer to FIG. 2).

The first filaments 5a, 5b, and 5c individually form the arch shapes as described above, thus making it possible to prevent damage from being done to an inner wall 100a of the blood vessel 100, resulting in the acquisition of the higher safety.

As shown in FIG. 2, the first filament 5a is positioned approximately on the plane (perpendicular to a paper of FIG. 2) passing through an extension line of a central axis 23 of the wire body 2. In other words, in the orientation shown in FIG. 2, the first filament 5a substantially lies on the extension line of the central axis 23 of the wire body 2. However, the first filament 5a is not necessarily oriented in the same direction as the extension line of the central axis of the wire body 2. In other words, the apex 51 of the first filament 5a is inclined toward the front side or back side of the paper plane in FIG. 2.

Further, the first filaments 5b, 5c are each inclined such that the distance from each filament to the central axis 23 of the wire body 2 increases in the forward direction toward the distal end side. In other words, in the side view of FIG. 2, the first filament 5b is inclined toward the left side and the first filament 5c is inclined toward the right side.

As described above, the first filaments 5b and 5c are each inclined with respect to the first filament 5a. Thus, the apex 51 of the first filament 5b becomes adjacent to the apex 51 of the first filament 5a through a gap 32, and the apex 51 of the first filament 5c becomes adjacent to the apex 51 of the first filament 5a through a gap 33. Thus, the foreign body capturing space 31 becomes large, thus making it possible to easily capture the embolus 200 into the foreign body capturing space 31.

As shown in FIG. 1, on the first filament 5b, there are provided the second filament 7a bridged between two branch points 54a located on the first filament 5b, and the second filament 7b bridged between two branch points 54b located on the first filament 5b.

The second filament 7a is provided so as to extend forward from one of the branch points 54a toward a distal end side, to turn rearward (toward a proximal end side) while forming a loop, and to return to the other branch point 54a. The second filament 7a projects into a left space of the first filament 5b in FIG. 2.

In approximately the same manner as the second filament 7a, the second filament 7b is provided so as to extend forward from one of the branch points 54b toward a distal end side, to turn rearward (toward a proximal end side) while forming a loop, and to return to the other branch point 54b. This second filament 7b projects into a right space (gap 32) of the first filament 5b shown in FIG. 2.

In other words, the second filaments 7a and 7b are individually formed into branch shapes of the first filament 5b, and are provided so as to fill spaces on both sides of the first filament 5b (refer to FIG. 2).

The second filaments 7a and 7b are not connected with the first filaments 5a and 5c. For example, in FIG. 2, the second filament 7b is adjacent to the first filament 5a but is spaced apart therefrom.

The second filament 7a is a first curved projection bent in a direction (first direction) deviated leftward in FIG. 2 from an extension line 52b of a central axis of a proximal end of the first filament 5b, in other words, in the direction opposite to the direction in which the gap 32 is formed, or the direction in which the second filament 7a gets away from the first filament 5a. The second filament 7b is a second curved projection bent in a direction (second direction) deviated rightward in FIG. 2 from the extension line 52b of the central axis of the proximal end of the first filament 5b, in other words, in the direction in which the second filament 7b enters the gap 32, or the direction in which the second filament 7b gets closer to the first filament 5a.

It is preferable that a sum of $\alpha$ and $\beta$ satisfy the following expression (1) in the natural state:

$$45° \leq \alpha+\beta \leq 90° \qquad (1)$$

where $\alpha$ is an angle made by the branch wire 4b and the first filament 5b in the distal end 41b of the branch wire portion 4b, and more specifically, an angle made by the extension line 42b of the central axis of the branch wire and the extension line 52b of the central axis of the proximal end of the first filament 5b, and $\beta$ is an angle made by the first filament 5b and the second filament 7a in the branch points 54a when viewed from the side (refer to FIG. 2), and more specifically, an angle made by the extension line 52b of the central axis of the proximal end of the first filament 5b and an extension line 72a of the central axis of the proximal end of the second filament 7a.

When the sum of $\alpha$ and $\beta$ is less than 45°, the foreign body capturing space 31 becomes small, and accordingly, there is a tendency to limit the size of the embolus 200 that is capturable within the space 31. Depending on the condition of the embolized or occuladed region, or the dimension and shape of the embolus, the embolus 200 may not be held securely, because the embolus 200 is not completely caught in the space. Meanwhile, when the sum of $\alpha$ and $\beta$ exceeds 90°, an apex 71 of the second filament 7a is directed toward the proximal end side, causing a risk of hindering an operation of inserting the wire 1 for removing an intravascular foreign body into the blood vessel 100 depending on the condition of the embolized or occuluded region, which is accordingly unpreferable. The possibility of causing damage to the inner wall of the blood vessel 100 is also increased.

It is more preferable that the sum of $\alpha$ and $\beta$ satisfy the following expression (2), and it is still more preferable that the sum of $\alpha$ and $\beta$ satisfy the following expression (3).

$$60° \leq \alpha+\beta \leq 80° \qquad (2)$$

$$60° \leq \alpha+\beta \leq 70° \qquad (3)$$

Note that the above-described relationships are also applied to an angle made by the branch wire 4b and the first filament 5c, and to an angle made by the first filament 5c and the second filament 7d.

Further, as shown in FIG. 1 (and also in FIG. 2), with regard to the branch points 54a of the second filament 7a and the branch points 54b of the second filament 7b, positions thereof on the first filament 5b substantially coincide with each other. Thus, in a process of manufacturing the wire 1 for removing an intravascular foreign body, the second filament 7a and the second filament 7b can be provided with good balance.

Note that the positions of the branch points 54a (also the branch points 54b, 54c, and 54d) are not particularly limited; however, when viewed from the side, for example, it is preferable that a value of T/T' be 1/20 to 19/20, and more preferable that the value of T/T' be 1/3 to 2/3, where T is a distance from the rear end of the first filament 5b (that is, the distal end 41b of the branch wire 4b) to the branch points 54a, and T' is a distance to the apex 51 of the first filament 5b from the rear end thereof (refer to FIG. 2).

Further, in approximately the same manner as the first filament 5b, on the first filament 5c, there are provided the second filament 7c bridged between two branch points 54c, and the second filament 7d bridged between two branch points 54d.

The second filament 7c is provided so as to extend forwardly from one of the branch points 54c toward a distal end side, to turn rearwardly toward a proximal end side while forming a loop, and to return to the other branch point 54c. As shown in FIG. 2, the second filament 7c projects into a left space (gap 33) of the first filament 5b in FIG. 2.

In approximately the same manner as the second filament 7c, the second filament 7d is provided so as to extend forward from one of the branch points 54d, to turn rearward while forming a loop, and to return to the other branch point 54d. This second filament 7d projects into a right space of the first filament 5b shown in FIG. 2.

In other words, the second filaments 7c and 7d are individually formed into branch shapes of the first filament 5c (refer to FIG. 2).

The second filaments 7c and 7d are not connected with the first filaments 5a and 5b. For example, in FIG. 2, the second filament 7c is adjacent to the first filament 5a but is spaced apart therefrom.

The second filament 7c is a first curved projection bent in a direction (first direction) deviated leftward in FIG. 2 from an extension line 52c of a central axis of a proximal end of the first filament 5c, in other words, in the direction in which the second filament 7c enters the gap 33, or the direction in which the second filament 7c gets closer to the first filament 5a. The second filament 7d is a second curved projection bent in a direction (second direction) deviated rightward in FIG. 2 from the extension line 52c of the central axis of the proximal end of the first filament 5c, in other words, in the direction opposite to the direction in which the gap 33 is formed, or the direction in which the second filament 7d gets away from the first filament 5a.

Further, as shown in FIG. 1 (and also in FIG. 2), with regard to the branch points 54c of the second filament 7c and the branch points 54d of the second filament 7d, positions thereof on the first filament 5c substantially coincide with each other. Thus, a substantially similar effect to the above-described effect brought by that the positions of the branch points 54a and 54b coincide with each other is obtained.

As described above, in the capturing portion 3, the first filaments 5a, 5b, and 5c are provided for the branch wires 4a and 4b. In a substantially similar way to this relationship, the second filaments 7a and 7b are provided for the first filament 5b, and the second filaments 7c and 7d are provided for the first filaments 5c.

In the capturing portion 3 (of the wire 1 for removing an intravascular foreign body) constituted as described above, for example, the embolus 200 can be surely prevented by the second filament 7b from slipping off from the gap 32 to a distal end (distal end) of the blood vessel 100 (for example, refer to FIG. 6). Thus, the capturing portion 3 can surely capture the embolus 200, and further, the embolus 200 once captured can be surely removed from the inside of the blood vessel 100.

Further, since a contact area of the capturing portion 3 with the embolus 200 in the capturing portion 3 concerned (foreign body capturing space 31) becomes large, the embolus 200 is surely clasped by the capturing portion 3, thus making it possible to surely capture and remove the embolus 200.

Note that the second filaments 7a, 7b, 7c, and 7d are individually composed of linear bodies, and are fixed (fixedly attached) to the first filaments 5b and 5c corresponding to the linear bodies. A method of fixing the second filaments to the first filaments is not particularly limited; however, for example, the proximal ends of the respective linear bodies can be individually fixed to the midway of the first filaments by performing braiding (winding), brazing, welding, adhesion using an adhesive, and so on.

Further, it is preferable that the second filaments 7a, 7b, 7c, and 7d be individually more flexible than the first filaments 5b and 5c. Thus, torque transmission and plunge capabilities of the capturing portion 3 can be ensured.

Further, an outer diameter $\varnothing$ D2 (refer to FIG. 2) of each of the second filaments 7a, 7b, 7c, and 7d (linear bodies) is not particularly limited; however, for example, in the case of capturing the embolus 200 (thrombus) in a cerebral vessel, usually, it is preferable that the diameter $\varnothing$ D2 be approximately 0,02 to 0,3 mm, and it is more preferable that the diameter $\varnothing$ D2 be approximately 0,02 to 0,1 mm.

Further, a length of each of the branch wires 4a and 4b is not particularly limited; however, for example, in the case of capturing the embolus 200 (thrombus) in the cerebral vessel, usually, it is preferable that the length be approximately 1,0 to 10, mm, and it is more preferable that the length be approximately 2,5 to 9,0 mm.

Further, an outer diameter $\varnothing$ d (refer to FIG. 2) of each of the branch wires 4a and 4b is not particularly limited; however, for example, in the case of capturing the embolus 200 (thrombus) in the cerebral vessel, usually, it is preferable that the diameter be approximately 0,04 to 0,5 mm, and it is more preferable that the diameter be approximately 0,06 to 0,2 mm.

Further, in the case of capturing the embolus 200 with a diameter of 7 mm, it is preferable that a length H (refer to FIG. 2) of the capturing portion 3 be 7 mm or more, and it is more preferable that the length H be 7 to 10 mm.

Further, an outer diameter $\varnothing$ D1 (refer to FIG. 2) of the first filament 5a, 5b, and 5c is not particularly limited; however, for example, in the case of capturing the embolus 200 (thrombus) in the cerebral vessel, usually, it is preferable that the diameter be approximately 0,05 to 0,5 mm, and it is more preferable that the diameter be approximately 0,1 to 0,4 mm.

Further, it is preferable that a radiopaque material be used as a constituent material of the capturing portion 3 (branch wires 4a and 4b, the first filaments 5a, 5b, and 5c, and the second filaments 7a, 7b, 7c, and 7d). Although the radiopaque material is not particularly limited, for example, there are mentioned gold, platinum, a platinum-iridium alloy, tungsten, tantalum, palladium, lead, silver, alloys and compounds which contain at least one of these, and the like.

By using the radiopaque material as described above, a capturing status of the embolus 200 in the capturing portion 3 can be easily confirmed in radioscopy using an X-ray and the like.

Further, it is preferable that the constituent material of the capturing portion 3 be an alloy which exhibits pseudoelasticity (including an alloy which exhibits superelasticity (hereinafter, referred to as "superelastic alloy")) in a living organism (at least at the temperature of the living organism (approximately 37°C)).

The alloy which exhibits the pseudoelasticity (hereinafter, referred to as "pseudoelastic alloy" includes alloys with any shapes represented by tensile stress-strain curves, both of alloys in which transformation points such as As, Af, Ms and Mf can be significantly measured and alloys in which the transformation points cannot be significantly measured, and all alloys which are deformed (strained) to a great extent by a stress and substantially return to original shapes thereof by removing the stress.

The pseudoelastic alloy includes the superelastic alloy. With regard to a preferable composition of the superelastic alloy, the superelastic alloy includes Ni-Ti-based alloys such as an Ni-Ti alloy in which Ni occupies 49 to 59 atomic %, a Cu-Zn alloy in which Zn occupies 38.5 to 41.5 wt%, a Cu-Zn-X alloy in which X occupies 1 to 10 wt% (X is at least one of Be, Si, Sn, A1, and Ga), an Ni-Al alloy in which A1 occupies 36 to 38 atomic %, and the like. Of those, an alloy with a particularly preferable composition is the above-described Ni-Ti-based alloys.

By using the pseudoelastic alloy as described above, the capturing portion 3 can obtain sufficient pliability, and stability against bending. Even if the capturing portion 3 repeats the deformation, the capturing portion 3 can be prevented from making a habit of being bent owing to excellent stability thereof.

The surface of the capturing portion 3 should be provided with anti-slipping means for preventing the embolus 200 which has been captured from slipping off from the capturing portion 3. Such anti-slipping means increases friction between the capturing portion 3 and the embolus 200, thereby allowing the capturing portion 3 to more surely hold (capture) the captured embolus 200.

The anti-slipping means is not particularly limited and may be formed by coating with an elastic material such as rubber having a comparatively high coefficient of friction or by sand blasting which produces fine rough surfaces (including irregular surfaces).

The outer surface of the capturing portion 3 may be provided with a coating layer as explained above for the wire body 2. The coating layer permits the capturing portion 3 to be inserted into and removed from the catheter 8 more smoothly.

As shown in FIG. 1, the first filament 5a has a plurality of projections 11 which project into the foreign body capturing space 31.

The method of forming the projections 11 is not particularly limited and may include winding one end of a number of flexible linear bodies (wires) around the first filament 5a, while allowing the other end to slightly project towards the capturing space 31.

The specific type material for forming each of the projections 11 is not particularly limited and may include various metallic materials or plastic materials which may be used alone or in combination with one another.

The length (on average) of each projection 11 is also not particularly limited and is preferably 0,1 to 5 mm and more preferably 0,5 to 2 mm.

As shown in FIG. 1, the first filament 5a also has a plurality of flexible fine fibers 12 projecting into the foreign body capturing space 31. Each fine fiber 12 should preferably be softer or more flexible than the projection 11.

The method of forming each of the fine fibers 12 is not particularly limited and may include, for example, a method involving winding a fibrous body around the first filament 5a or attaching fine fibers by static flocking.

The specific type of fine fibers 12 is not particularly limited and may be formed from any material including radiotransparent fibers such as Dacron (polyester), polyglycolic acid, polylactic acid, fluoropolymer (polytetrafluoroethylene), nylon (polyamide), cotton, and silk. Other materials may include metallic yarn coated with radiotransparent fiber or radiopaque fiber.

The length (on average) of each fine fiber 12 is also not particularly limited and is preferably 0,1 to 5 mm in length, more preferably 0,5 to 3 mm.

The projections 11 and the fine fibers 12 formed as mentioned above and directed into the capturing space 31 make it possible to relatively surely capture the embolus 200. In the case of a comparatively hard embolus 200, the projections 11 stab the embolus 200, thereby inhibiting or preventing it from slipping out from the foreign body capturing space 31. In the case of a comparative soft embolus 200, the fine fibers 12 hang on to the embolus 200, thereby inhibiting or preventing it from slipping off from the foreign body capturing space 31.

The projections 11 may be formed not only on the first filament 5a but also, for example, on the first filaments 5b, 5c, the second filaments 7a, 7b, 7c, or 7d, and may be formed on any one or more of those regions.

The fine fibers 12 may be formed not only on the first filament 5a but also, for example, on the first filaments 5b, 5c, the second filaments 7a, 7b, 7c, or 7d, and may be formed on any one or more of those regions.

Further, in this embodiment, the branch wires 4a and 4b and the first filaments 5a, 5b and 5c are formed of the continuous loop wires 6a, 6b and 6c; however, in the present invention, the branch wires 4a and 4b and the first filaments 5a, 5b and 5c may be formed by connecting (coupling) separate members to one another. In this case, a method of fixing the first filaments 5a, 5b and 5c to the branch wires 4a and 4b may be any method, which includes, for example, brazing, welding, and adhesion using an adhesive.

Further, the loop wires 6a, 6b and 6c equivalent to the portions constituting the branch wires 4a and 4b do not have to be stranded as in this embodiment, and may simply be in a collected state (bundled state).

A medical instrument 9 according to the present invention includes the wire 1 for removing an intravascular foreign body together with the catheter 8 in which the lumen 82 is formed.

An example of one way of using the wire 1 for removing an intravascular foreign body is described in the following.

[1] FIG. 3 shows a state of a vessel 100 which is clogged with an embolus 200 (such as a thrombus) which hinders blood flow. The embolus 200 is almost immobile because the embolus 200 is pushed against the inner wall 100a of the vessel 100 by blood pressure.

The catheter (microcatheter) 8 and the guide wire 10 (which has been passed through the lumen 82 of the catheter 8) are inserted into the vessel 100. Next, a distal end 101 of the guide wire 10 projects from the forward open end 81 of the catheter 8 beyond the embolus 200 (toward a peripheral side). In other words, this is a state in which the distal end 101 of the guide wire 10 passes through the gap between the embolus 200 and the inner wall 100a of the vessel 100 and moves beyond the embolus 200. This operation can be relatively easily accomplished by using a micro-guide wire which has good lubricity as the guide wire 10.

[2] After the distal end 101 of the guide wire 10 has moved past the embolus 200, the catheter 8 is advanced with respect to the guide wire 10 so that the distal end of the catheter 8 moves into the gap between the embolus 200 and the inner wall 100a of the vessel 100 as shown in FIG. 4. This operation can be easily accomplished because the distal end of the catheter 8 smoothly moves along the guide wire 10 into the gap.

In the conventional therapy, a thrombolytic agent is injected retrogradely through the catheter 8 to accelerate thombolysis. However, in doctors' experience, there are thrombi which are not dissolved by a thrombolytic agent often or dissolution by a thrombolytic agent takes a long time. The present invention is effective in such a case.

[3] From the state shown in FIG. 4, the guide wire 10 is removed, and the wire 1 for removing an intravascular foreign body according to the present invention is inserted into the lumen 82 of the catheter 8. At this time, as shown in FIG. 5, the capturing portion 3 is housed in the lumen 82, and the distal end 41a of the branch wire 4a and the distal end 41b of the branch wire 4b are turning into a state of being regulated by an inner wall surface 821 which defines the lumen 82, and being approaching each other. To be specific, an interval p is smaller than in the natural state (refer to FIG. 1) (hereinafter, this state is referred to as "contracted state"). Further, the apices of the first filaments 5a to 5c and the second filaments 7a to 7d approach one another (come close to one another). Here, the "natural state" refers to a state where an external force is not applied to the branch wires 4a and 4b.

[4] When the capturing portion 3 which has stayed in the catheter 8 in the contracted state is allowed to project from the distal end opening 81 (refer to FIG. 6) by elasticity of its own, the distal end portion 41a and the distal end portion 41b apart from each other.

To be specific, the interval p becomes large. Further, at this time, the apices of the first filaments 5a to 5c and the second filaments 7a to 7d are spaced apart from one another. As a result, the capturing portion 3 turns to the natural state. By the foreign body capturing space 31 in the state as described above, the embolus 200 can be captured surely (easily).

[5] From the state as described above, where the capturing portion 3 is allowed to project from the distal end opening 81 of the catheter 8, the catheter 8 is slightly moved rearward toward a proximal end side, and the distal end of the catheter 8 is pulled back to the front of the embolus 200. Then, as shown in FIG. 6, the embolus 200 is captured (housed) in the foreign body capturing space 31 of the capturing portion 3 in a manner of being scooped. In other words, the embolus 200 enters the foreign body capturing space 31 from an upper side shown in FIG. 6. A distal end of the embolus 200 which has entered the foreign body capturing space 31 is surely covered with the first filaments 5a to 5c and the second filaments 7a to 7d. Thus, the embolus 200 is surely prevented from leaving the capturing portion 3 (foreign object capturing space 31), particularly, to a distal end of the blood vessel 100.

[6] When the embolus 200 is housed in the capturing portion 3, the wire body 2 is pulled with respect to the catheter 8 rearward toward the proximal end. Thus, the proximal ends 42 of the branch wires 4a and 4b abut on (an edge of) the distal end opening 81, and are drawn into the catheter 8 while narrowing the interval therebetween, and the loop formed of the branch wires 4a and 4b (loop wires 6a, 6b and 6c) becomes small. Hence, the embolus 200 is tightened by the branch wires 4a and 4b.

[7] The wire 1 for removing an intravascular foreign body and the catheter 8 are then removed together from the vessel, while maintaining the tightened state. Thus, the embolus 200 is eliminated and captured in the guiding catheter or sheath introducer (not shown).

It is noted that when the tightening operation described in the paragraph [6] is not performed, but the embolus 200 is housed in the capturing portion 3, the wire 1 for removing an intravascular foreign body may be removed out together with the catheter 8 while keeping such a housed state, and thus the embolus 200 may be removed.

Further, it is preferable that the interval p in the contracted state be 0,53 mm (0.021 inch) or less, and more preferably 0,45 mm (0.018 inch) or less.

<Second Embodiment>

FIG. 7 is a perspective view showing a second embodiment of the wire for removing an intravascular foreign body according to the present invention (in the natural state), and FIG. 8 is a side view of the wire for removing an intravascular foreign body, which is shown in FIG. 7.

Hereinafter, with reference to the drawings, the second embodiment of the wire for removing an intravascular foreign body according to the present invention is described. Differences from the first embodiment are mainly discussed and points which are the same as those associated with the first embodiment will not be repeated.

This embodiment is similar to the first embodiment except that the arrangement positions of the second filaments with respect to the first filaments are different.

As shown in FIG. 7 and FIG. 8, a wire 1A for removing an intravascular foreign body according to this embodiment includes second filaments 7e and 7f branching from the midway of the first filament 5b, and second filaments 7g and 7h branching from the midway of the first filament 5c.

The second filament 7e is bridged between two branch points 54e. Further, the second filament 7f is bridged between two branch points 54f.

The positions of the branch points 54e and the branch points 54f on the first filament 5b are different from each other. To be specific, the branch points 54f are located closer to the distal end with respect to the branch points 54e. Thus, the sizes of the respective loops of the second filaments 7e and 7f can be differentiated from each other, and therefore, the second filaments 7e and 7f, for example, corresponding to the size of the embolus 200 can be provided.

Further, as shown in FIG. 8, the apex 71 of the second filament 7f is located the distal to (on the distal side than) the apex 51 of the first filament 5b. Thus, the foreign body capturing space 31 in this embodiment can be set larger than the foreign body capturing space 31 of the above-described first embodiment, and therefore, the embolus 200 can be housed in the foreign body capturing space 31 more easily.

Further, the second filament 7g is bridged between two branch points 54g. Further, the second filament 7h is bridged between two branch points 54h.

The positions of the branch points 54g and the branch points 54h are different from each other. To be specific, the branch points 54g are located distal to (on the distal end side than) the branch points 54h. Thus, a substantially similar effect to the above-described effect brought by the positions of the branch points 54e and the branch points 54f being different from each other is obtained.

<Third Embodiment>

FIG. 9 is a perspective view showing a third embodiment of the wire for removing an intravascular foreign body according to the present invention (in the natural state), and FIG. 10 is a side view of the wire for removing an intravascular foreign body, which is shown in FIG. 9.

Hereinafter, with reference to those drawings, the third embodiment of the wire for removing an intravascular foreign body will be described. It is noted that the description will be made mainly of differences from the second embodiment and points which are the same as those associated with the second embodiment will not be repeated.

This embodiment is similar to the second embodiment except that third filaments are provided.

As shown in FIG. 9 and FIG. 10, in the capturing portion 3 of a wire 1B for removing an intravascular foreign body according to this embodiment, third filaments 13a to 13f branching from the midway of the second filaments 7e to 7h are provided on the second filaments 7e to 7h, respectively.

On the second filament 7e, there are provided the third filament 13a bridged between two branch points 131a, and the third filament 13b bridged between two branch points 131b.

The third filament 13a is provided so as to extend forward from one of the branch points 131a toward a distal end side, to turn rearward toward a proximal end side while drawing a loop, and to return to the other branch point 131a. As shown in FIG. 10, the third filament 13a projects into a lower side space of the second filament 7e in FIG. 10.

The third filament 13a is preferably a fiber-like element having more flexibility than the first filament 6b and the second filament 7e. The materials referred to above for the fine fibers 12 can be used to produce such fiber-like filament.

In approximately the same manner as the third filament 13a, the third filament 13b is provided so as to extend forward from one of the branch points 131b, to turn rearward while drawing a loop, and to return to the other branch point 131b. This third filament 13b projects into an upper side space of the second filament 7e in the FIG. 10.

Further, the positions of the branch points 131a and the branch points 131b on the second filament 7e are different from each other. To be specific, the branch points 131a are located closer to the apex 71 of the second filament 7e than the branch points 131b. Thus, the sizes of the respective loops of the third filaments 13a and 13b can be differentiated from each other, and therefore, the second filaments 7e and 7f, for example, corresponding to the size of the embolus 200 can be provided.

On the second filament 7f, there are provided the third filament 13c bridged between two branch points 131c.

The third filament 13c is provided so as to extend forward from one of the branch points 131c toward a distal end side, to turn rearward toward a proximal end side while drawing a loop, and to return to the other branch point 131c. As shown in FIG. 10, the third filament 13c projects into a left space of the second filament 7f in FIG. 10.

On the second filament 7g, there are provided the third filament 13d bridged between two branch points 131d.

The third filament 13d is provided so as to extend forward from one of the branch points 131d toward a distal end side, to turn rearwardly toward a proximal end while drawing a loop, and to return to the other branch point 131d. As shown in FIG. 10, the third filament 13d projects into a left space of the second filament 7g in FIG. 10.

On the second filament 7h, there are provided the third filament 13e bridged between two branch points 131e, and the third filament 13f bridged between two branch points 131f.

The third filament 13e is provided so as to extend forward from one of the branch points 131e toward a distal end side, to turn rearwardly toward a proximal end side while drawing a loop, and to return to the other branch point 131e. As shown in FIG. 10, the third filament 13e projects into an upper space of the second filament 7h in FIG. 10.

In approximately the same manner as the third filament 13e, the third filament 13f is provided so as to extend forward from one of the branch points 131f, to turn rearward while drawing a loop, and to return to the other branch point 131f. This third filament 13f projects into a lower side space of the second filament 7h.

Further, the positions of the branch points 131e and the branch points 131f are different from each other. To be specific, the branch points 131e are located closer to the apex 71 of the second filament 7h than the branch points 131f. Thus, a substantially similar effect to the above-described effect brought by the positions of the branch points 131a and the branch points 131b being different from each other is obtained.

The third filaments 13a to 13f constituted as described above are provided, thus making it possible to cover a distal end of the captured embolus 200 more securely. Thus, the embolus 200 can be more surely prevented from leaving the capturing portion 3 (foreign object capturing space 31), particularly, to the distal end of the blood vessel 100. Hence, the wire 1B for removing an intravascular foreign body can capture and remove the embolus 200 more surely.

Note that an average outer diameter $\varnothing$ D3 (see FIG. 10) of the third filaments 13a, 13b, 13c, 13d, 13e and 13f is not particularly limited. However, for example, in the case of capturing the embolus 200 (thrombus) in the cerebral vessel, usually, it is preferable that the diameter $\varnothing$ D3 be approximately 0,02 to 0,2 mm, and more preferably approximately 0,02 to 0,1 mm.

A description of a second configuration of the wire for removing an intravascular foreign body according to the present invention will be made below.

The second configuration of the wire for removing an intravascular foreign body according to the present invention is characterized in that at least one of the filaments includes a plurality of bent or steeply curved inflection points, and coupling portions each of which is located between two of the inflection points and disposed so as to enter a space between the filaments adjacent to each other.

In the following seven comparative examples for better understanding the invention will be described.

First comparative example

FIG. 11 is a perspective view showing a first comparative example of the wire for removing an intravascular foreign body (in the natural state), FIG. 12 is a side view of the wire for removing an intravascular foreign body, which is shown in FIG. 11, and FIG. 13 is a view showing a state where the embolus in the blood vessel is captured using the wire for removing an intravascular foreign body, which is shown in FIG. 12.

Hereinafter, with reference to the drawings, the fourth embodiment of the wire for removing an intravascular foreign body will be described. It is noted that the description will be made mainly of differences from the first embodiment and points which are the same as those associated with the first embodiment will not be repeated.

As shown in FIG. 11, in a wire 1C for removing an intravascular foreign object according to the present invention in the first comparative example, the capturing portion 3 is composed of the two branch wires 4a and 4b branching from the distal end of the wire body 2, and a plurality (three in this comparative example) of filaments 14a, 14b and 14c bridged between the branch wire 4a and the branch wire 4b.

The branch wire 4a is composed of a stranded wire formed by stranding and integrally collecting portions of the filaments 14a, 14b and 14c on one proximal end side. Further, in a substantially similar way to the branch wire 4a, the branch wire 4b is composed of a stranded wire formed by stranding and integrally collecting portions of the filaments 14a, 14b and 14c on the other proximal end side.

As show in FIG. 11, between the distal end 41a of the branch wire 4a and the distal end 41b of the branch wire 4b, the three filaments 14a, 14b and 14c which form linear shapes are provided so as to be bridged therebetween.

In the filaments 14a, 14b and 14c, tip(distal)-end portions (center portions) thereof are spaced apart from one another in the natural state. To be specific, as shown in FIG. 12, when viewed from a side, the filament 14a is formed on the central axis 23 of the wire body 2, and the filaments 14b and 14c adjacent to the filament 14a are bent (or curved) toward outside (left and right sides in FIG. 12), respectively.

As shown in FIG. 11, each of the filaments 14b and 14c is formed (provided) to extend forward from a distal end of the branch wire 4a toward the distal end side, to turn rearward toward a proximal end side while being curved in one direction (while forming an arch shape), and to return to the distal end of the branch wire 4b.

The wire 1C for removing an intravascular foreign object includes the filament 14a having a bent and deformed portion 15a.

The bent and deformed portion 15a is formed in such a manner that a part (center portion) of the filament 14a is bent or curved irregularly, that is, in many directions. In other words, the bent and deformed portion 15a is composed of many (plurality of) inflection points 16, and many coupling portions 17 each of which is located between adjacent two inflection points.

The respective inflection points 16 are portions where the filament 14a is partially bent or steeply curved. Further, as shown in FIG. 12, the respective inflection points 16 are scattered (located) in a gap (space) 32 between the filament 14a and the filament 14b and a gap (space) 33 between the filament 14a and the filament 14c.

Further, as shown in FIG. 11 and FIG. 12, the many coupling portions 17 are arranged to enter the gap 32 and the gap 33. In other words, the coupling portions 17 adjacent to each other extend in directions different from each other. To be specific, the many coupling portions 17 extend in many directions.

The shape of the proximal end of the filament 14a having the bent and deformed portion 15a is as follows: A portion 14a1 close to the proximal end of the filament 14a is positioned approximately on the plane (perpendicular to a paper of FIG. 12) passing through the extension line of the central axis 23 of the wire body 2. In the orientation shown in FIG. 12, the portion 14a1 substantially lies on the extension line of the central axis 23 of the wire body 2. Therefore, in FIG. 12, the central axis of the portion 14a1 close to the proximal end of the filament 14a and its extension line are represented by the extension line of the central axis 23 of the wire body 2. However, this portion 14a1 is not necessarily oriented in the same direction as the extension line of the central axis 23 of the wire body 2. In other words, the distal end side of the portion 14a1 is inclined toward the frond side or back side of the paper plane in FIG. 12.

A portion 14a2 of the filament 14a which is distal to the portion 14a1 is bent in a direction (first direction) deviated rightward in FIG. 12 from the portion 14a1, in other words, in the direction in which the portion 14a2 enters the gap 33, or the direction in which the portion 14a2 gets closer to the filament 14c. The portions 14a1 and 14a2 preferably form an angle $\gamma$ of not more than 90°.

A portion 14a3 of the filament 14a which is distal to the portion 14a2 is bent in a direction (second direction) deviated leftward in FIG. 12 contrary to the portion 14a2, in other words, in the direction in which the portion 14a3 enters the gap 32, or the direction in which the portion 14a3 gets closer to the filament 14b.

The portion 14a2 that forms an angle $\gamma$ with respect to the portion 14a1 is substantially not bent and has an approximately linear shape, but may have a slightly curved shape.

The bent and deformed portion 15a constituted as described above is provided, and thus volumes of the gap 32 and the gap 33, which are filled with the filament 14a, become larger than in the case where the filament 14a is constituted, for example, in a substantially similar way to the filaments 14b and 14c, that is, where the filament 14a is formed into the arch shape. To be specific, void volumes of the gap 32 and the gap 33 are reduced. Thus, the embolus 200 which has entered (has been housed in) the capturing portion 3 can be surely prevented from slipping off from (leaving) the gap 32 and the gap 33, for example, owing to the blood flow to the distal end (tip end) side and the operation of the wire 1C for removing an intravascular foreign body in the proximal (rearward) direction. Therefore, the embolus 200 can be surely captured and removed (see FIG. 13). Here, the term "void volume" refers to a ratio, to the gap 32 (also the gap 33), of a portion except the portion occupied by the bent and deformed portion 15a, that is, of a void portion.

Further, it is preferable that, in the filament 14a, the bent and deformed portion 15a be composed of a linear body having portions different in thickness. Thus, for example, relatively thin portions are defined as the inflection points 16, and relatively thick portions as the coupling portions 17, thus making it possible to easily form the bent and deformed portion 15a. Further, the relatively thick portions are defined as the coupling portions 17, thus making it possible to further reduce the void volumes more. Thus, the embolus 200 in the capturing portion 3 can be more surely prevented from slipping off from the gap 32 and the gap 33. Therefore, the embolus 200 can be captured and removed more surely.

Further, it is preferable that the bent and deformed portion 15a be superior in pliability than the branch wires 4a and 4b. Thus, for example, when the embolus 200 is captured by the capturing portion 3, the bent and deformed portion 15a can be deformed to fit to the shape of the embolus 200 in the capturing portion 3, and therefore, the embolus 200 can be captured and removed more surely. Further, the torque transmission and plunge capabilities (pushability) of the capturing portion 3 can be ensured.

Further, in the bent and deformed portion 15a, the many coupling portions 17 are arranged uniformly to fill the gaps 32 and 33 evenly. To be specific, it is preferable that the bent and deformed portion 15a be formed so as not to cause unevenness in the void volumes of the gaps 32 and 33. Thus, it is made possible to prevent, in the capturing portion 3, an occurrence of the portions where the embolus 200 housed in the capturing portion 3 is less prone to slip off from the gaps 32 and 33, and portions where the embolus 200 is prone to slip off therefrom.

Second comparative example

FIG. 14 is a perspective view showing a second comparative example of the wire for removing an intravascular foreign body (in the natural state), and FIG. 15 is a side view of the wire for removing an intravascular foreign body, which is shown in FIG. 14.

Hereinafter, with reference to the drawings, the second comparative example of the wire for removing an intravascular foreign body will be described. It is noted that the description will be mainly of differences from the first comparative example and points which are the same as those associated with the first comparative example will not be repeated.

This comparative example is similar to the first comparative example except that the shapes (constructions) of the both outside filaments arranged on the peripheral side of three filaments, respectively, are different.

In the wire 1D for removing an intravascular foreign body, which is shown in FIG. 14 and FIG. 15, not only the bent and deformed portion 15a is provided on the filament 14a, but also bent and deformed portions 15b and 15c, which are substantially the same as the bent and deformed portion 15a, are provided on the filaments 14b and 14c, respectively.

As described above, each construction of the bent and deformed portions 15b and 15c is substantially the same as to the construction of the bent and deformed portion 15a, and accordingly, a description thereof will be omitted.

Incidentally, in this comparative example, the bent and deformed portions 15a, 15b and 15c are provided on the filaments 14a, 14b and 14c, respectively. Thus, the volumes of the gaps 32 and 33 filled with the filaments concerned, become larger. To be specific, the void volumes can be reduced more. Hence, the embolus 200 housed in the capturing portion 3 can be more surely prevented from slipping off from the gap 32 and the gap 33 in the distal (forward) direction of the blood vessel 100, and therefore, the embolus 200 can be captured and removed more surely.

Further, as shown in FIG. 15 (and also in FIG. 14), in the natural state, the bent and deformed portion 15a is located distal to (on the distal end side than) the bent and deformed portions 15b and 15c. Thus, in the contracted state of the capturing portion 3, the bent and deformed portion 15a is located distal to (on the distal end side than) the bent and deformed portions 15b and 15c, thus making it possible to prevent intertwinement of the bent and deformed portions 15a, 15b and 15c owing to complicated (intricate) shapes of the bent and deformed portions 15a, 15b and 15c.

Further, the bent and deformed portion 15a just needs to be located distal to the distal end than the bent and deformed portions 15b and 15c in the contracted state of the capturing portion 3. A construction only needs to be made so that the bent and deformed portion 15a is located on the distal end side in the contracted state even if the bent and deformed portion 15a is located at substantially the same position as those of the bent and deformed portions 15b and 15c in the longitudinal direction of the wire 1D for removing an intravascular foreign body in the natural state.

Third comparative example

FIG. 16 is a side view of a third comparative example of the wire fore removing an intravascular foreign body (in the natural state).

Hereinafter, with reference to the drawings, the third comparative example of the wire for removing an intravascular foreign body will be described. It is noted that the description will be made mainly on differences from the previous embodiments and comparative examples, and points which are the same as those associated with the previous embodiments and comparative examples will not be repeated.

This comparative example is similar to the second comparative example except that a positional relationship among the bent and deformed portions respectively provided on the three filaments is different.

In a wire 1E for removing an intravascular foreign body, which is shown in FIG. 16, the bent and deformed portion 15a is located proximal to (on the proximal end side than) the bent and deformed portions 15b and 15c in the natural state. Moreover, the wire 1E for removing an intravascular foreign body is constituted such that the bent and deformed portion 15a is located proximal to (on the proximal end side than) the bent and deformed portions 15b and 15c even in the contracted state of the capturing portion 3. Further, the intertwinement of the bent and deformed portions 15a, 15b and 15c can be prevented, which may by caused by the complicated (intricate) shapes of the bent and deformed portions 15a, 15b and 15c.

However, the bent and deformed portion 15a is located distal to the bent and deformed portions 15b and 15c, and thus, when the size of the center filament 14a is set equal to the case where the bent and deformed portion 15a is located proximal to (on the proximal end side than) the portions 15b and 15c, the lengths and widths of the filaments 14b and 14c in the longitudinal direction can be suppressed as compared with the above-described case. Accordingly, the filaments 14b and 14c on both sides can be set small. Therefore, the capturing portion 3 can be reduced in size while ensuring the size of the foreign object capturing space 31.

It is noted that the bent and deformed portion 15a only needs to be located proximal to the bent and deformed portions 15b and 15c in the contracted state of the capturing portion 3. The construction should be made so that even if the bent and deformed portion 15a is located at substantially the same position as the bent and deformed portions 15b and 15c in the longitudinal direction of the wire 1E for removing an intravascular foreign body in the natural state, the bent and deformed portion 15a is located on the proximal end side in the contracted state.

Fourth comparative example

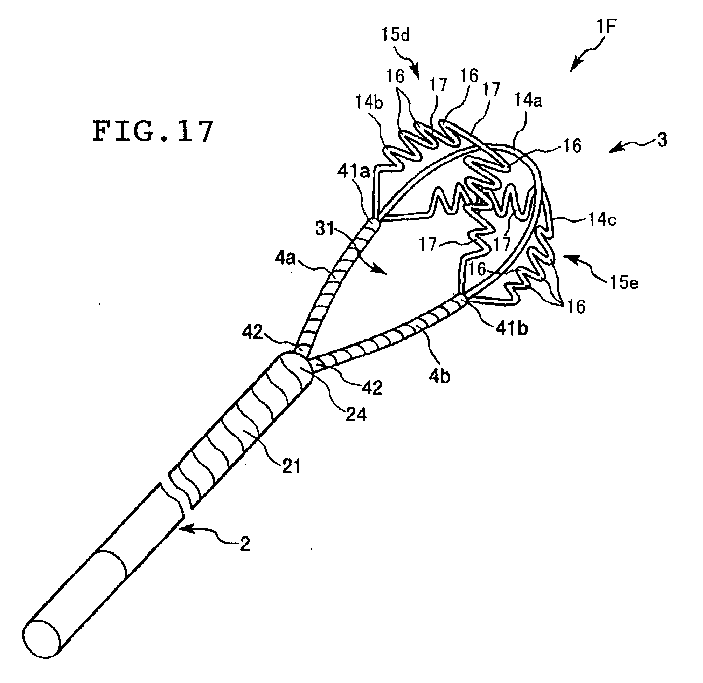

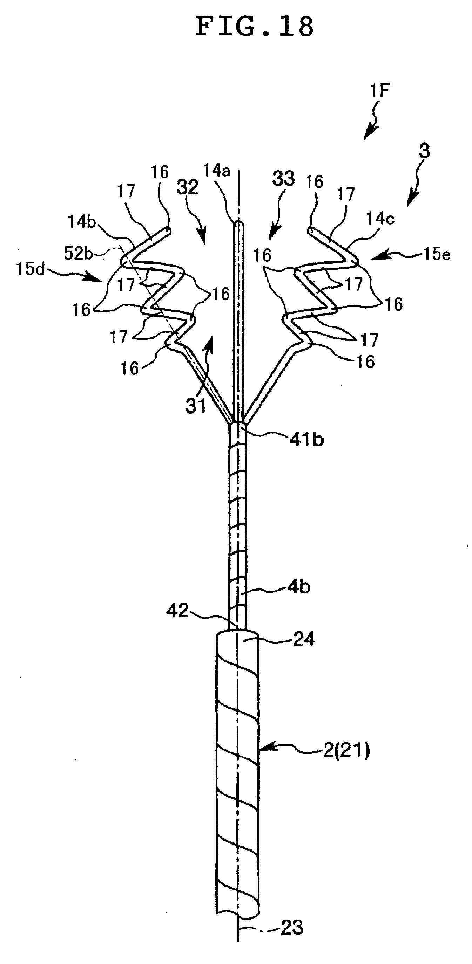

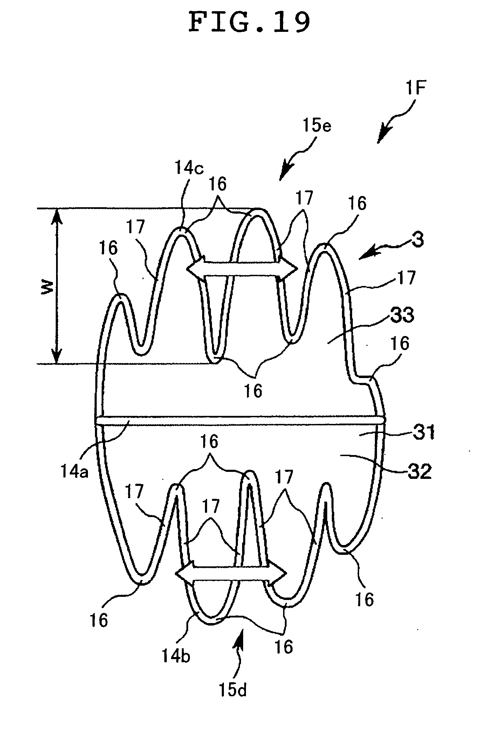

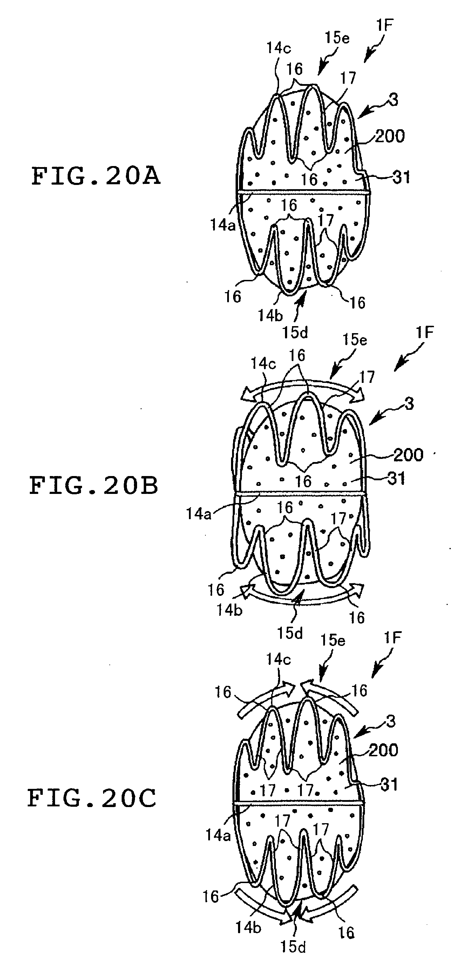

FIG. 17 is a perspective view showing a fourth comparative example of the wire for removing an intravascular foreign body (in the natural state), FIG. 18 is a side view of the wire for removing an intravascular foreign body, which is shown in FIG. 17, FIG. 19 is a view (plan view) of the wire for removing an intravascular foreign body, which is shown in FIG. 17, from the distal end side, and FIGS. 20A to 20C are views (plan views) showing a state where the capturing portion of the wire for removing an intravascular foreign body, which is shown in FIG. 17, captures the embolus.

Hereinafter, with reference to the drawings, the fourth comparative example of the wire for removing an intravascular foreign body will be described. It is noted that the description will be mainly made of differences form the previous embodiments and comparative examples, and points which are the same as those associated with the previous embodiments and comparative examples will not be repeated.

This comparative example is similar to the second comparative example except that the shapes of the bent and deformed portions are different.

In the capturing portion 3 of a wire 1F for removing an intravascular foreign body, which is shown in FIGS. 17 to 19, the filament 14a forms the arch shape, and the filaments 14b and 14c have bent and deformed portions 15d and 15e, respectively.

Note that, the constructions of the filaments 14b and 14c are substantially similar to those of the filaments 14b and 14c of the fifth embodiment, so a description thereof will be omitted. Further, the shapes of the bent and deformed portion 15d and the bent and deformed portion 15e are substantially the same, so a description will be made of the bent and deformed portion 15d representatively.

Unlike the one which forms the irregular shape, such as the bent and deformed portion 15a of the fifth embodiment, the bent and deformed portion 15d forms a regular shape. As a typical example of the regular shape, the shape of the bent and deformed portion 15d of this embodiment can be made into a wave shape when the bent and deformed portion 15d concerned is viewed from the distal end side of the wire body 2 in the longitudinal direction (refer to FIG. 19).

In the wire 1F for removing an intravascular foreign body, the shape of the bent and deformed portion 15d is the wave shape. Thus, there exist alternately the inflection points 16 bent so as to deviate from the extension line 52b of the central axis of the proximal end portion of the filament 14b to the left side in FIG. 18 (first direction), and the inflection points 16 bent so as to deviate from the extension line 52b of the central axis of the proximal end portion of the filament 14b to the right side in FIG. 18 (second direction).

As shown in FIG. 19, in the bent and deformed portion 15d, each of the inflection points 16 is formed so as to correspond to a crest portion or trough (valley) portion of the wave shape (wave).

For example, when the bent and deformed portion 15d constituted as described above (which forms the wave shape) is superior in pliability in a similar way to the bent and deformed portion 15a of the second comparative example of the present invention, the bent and deformed portion 15d can easily expand and contract in directions (directions indicated by arrows of FIG. 19) in which the inflection points 16 adjacent to each other approach and are spaced apart from each other. Further, the bent and deformed portion 15d expands and contracts to some extent also in directions perpendicular to the arrow directions of FIG. 19.

Thus, for example, when the embolus 200 is captured by the capturing portion 3, the bent and deformed portion 15d can be deformed so as to fit the shape of the embolus 200 in the capturing portion 3. Therefore, the embolus 200 can be captured and removed more surely.

Further, in the bent and deformed portion 15d, amplitude w thereof is gradually increased from the proximal end side (from the branch wires 4a and 4b) to the distal end side (apex). In a region where the amplitude w of the bent and deformed portion 15d is large, it becomes easy for the bent and deformed portion 15d to expand and contract. To be specific, the pliability of the bent and deformed portion 15d is increased. Further, in a region where the amplitude w of the bent and deformed portion 15d is small, the expansion and contraction thereof are suppressed. That is, the pliability is decreased.

Hence, with regard to the bent and deformed portion 15d, the pliability thereof is gradually increased (changed) from the proximal end side to the distal end side, that is, along the expansion and contraction directions.

A description will be made of a process in which the capturing portion 3 having the bent and deformed portion 15d as described above (and also the bent and deformed portion 15e) captures and removes the embolus 200 with reference to FIGS. 20A to 20C.

By substantially similar operations to those described in the paragraphs [1] to [5] of the method of operating the wire 1 for removing an intravascular foreign body according to the first embodiment of the present invention, the embolus 200 is housed in the foreign object capturing space 31 of the capturing portion 3.

When the wire body 2 is slightly pulled rearward (toward the proximal end side) in a state (state shown in FIG. 20A) where the embolus 200 is housed in the capturing portion 3, as shown in FIG. 20B, the embolus 200 does not move, but the bent and deformed portions 15d and 15e are pulled by the branch wires 4a and 4b connected to the wire body 2, respectively, and extend in arrow directions shown in FIG. 20B. The bent and deformed portions 15d and 15e which have expanded covers the embolus 200 along the shapes of the embolus 200.

After that, as shown in FIG. 20C, the bent and deformed portions 15d and 15e individually contract instantaneously in arrow directions shown in FIG. 20C by their own elasticity. Thus, the embolus 200 is tightened (sandwiched). To be specific, the embolus 200 is compressed and captured.

After that, by a substantially similar operation to that described in the paragraph [7] of the method of operating the wire 1 for removing an intravascular foreign body according to the first embodiment, the embolus 200 can be removed.

With such the construction, the embolus 200 is tightened and held with a force which is relatively weak, and is also compressed and held, and thus the embolus 200 can be surely removed without being crushed (broken) when the wire body 2 (of the wire 1F for removing an intravascular foreign body) is pulled rearward.

Fifth comparative example

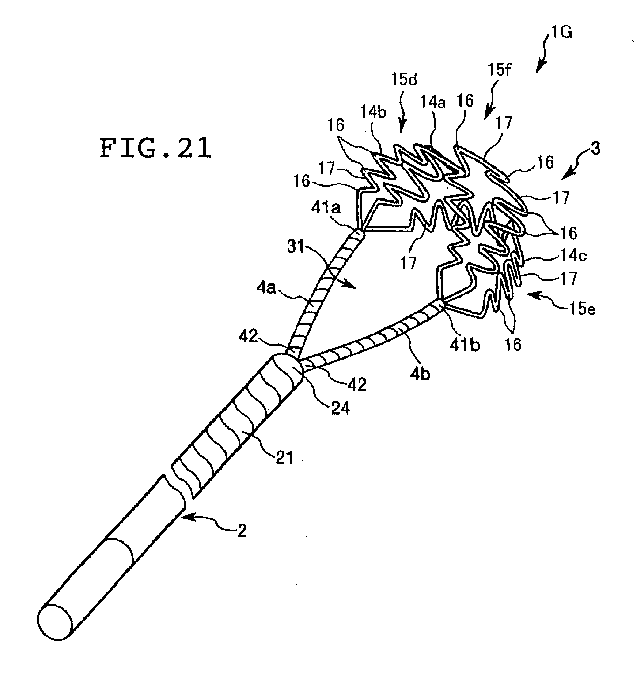

FIG. 21 is a perspective view of a wire for removing an intravascular foreign body according to a fifth comparative example (in a natural state).

Hereinafter, with reference to the drawings, the fifth comparative example of the wire for removing an intravascular foreign body will be described. It is noted that the description will be made of differences from the embodiments and comparative example described above, and points which are the same as those associated with the previous embodiments and comparative example will not be repeated.

This comparative example is similar to the fourth comparative example except that the shape (constructions) of the filament arranged on the center of the three filaments is different from the other.

In the capturing portion 3 of a wire 1G for removing an intravascular foreign body, which is shown in FIG. 21, not only the bent and deformed portions 15d and 15e which form the wave shapes are provided on the filaments 14b and 14c, respectively, but also a bent and deformed portion 15f substantially similar to the bent and deformed portions 15d and 15e is provided on the filament 14a.

Note that, the construction (shape) of the bent and deformed portion 15f is a substantially similar to those of the bent and deformed portions 15d and 15e, a description thereof will be omitted.

The bent and deformed portion 15f and the bent and deformed portion 15d are spaced apart from each other to an extent where each of the crest portions (inflection points 16) of the bent and deformed portion 15f does not enter the space between the two adjacent coupling portions 17 of the bent and deformed portion 15d, that is, each of the trough portions (inflection points 16). Further, the bent and deformed portion 15f and the bent and deformed portion 15e are spaced apart from each other to an extent where the respective crest portions of the bent and deformed portion 15f do not enter the respective trough portions of the bent and deformed portion 15e.

In the capturing portion 3 constituted as described above, the void volumes of the gaps 32 and 33 are set smaller. Therefore, the embolus 200 housed in the capturing portion 3 can be more surely prevented from leaving the gap 32 and the gap 33. Thus, the embolus 200 can be captured and removed more surely.

Sixth comparative example

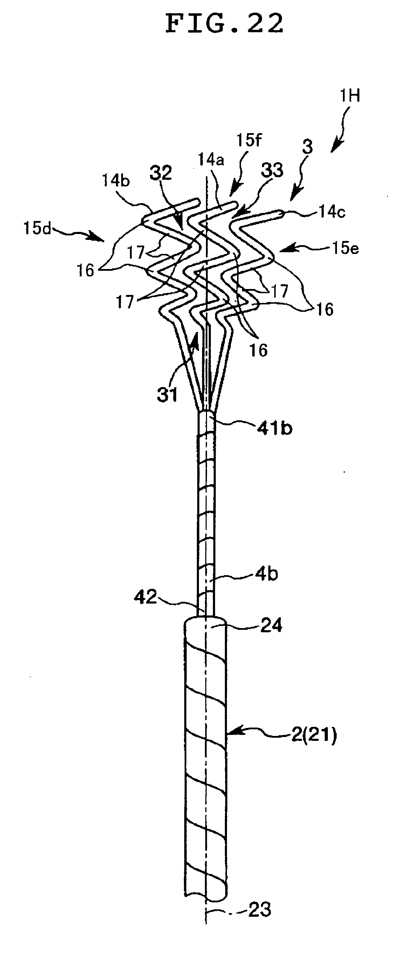

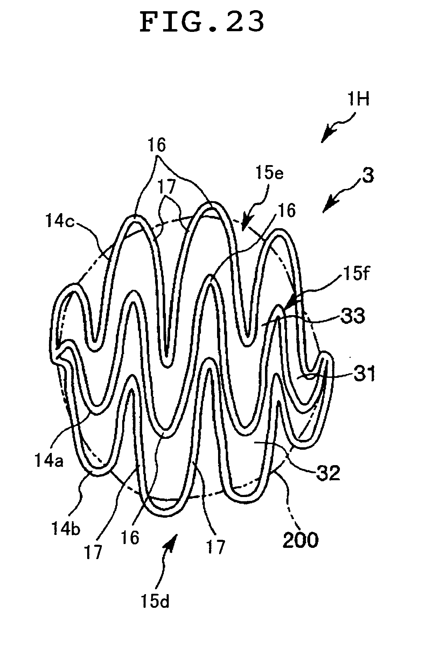

FIG. 22 is a side view showing a wire for removing an intravascular foreign object according to a sixth comparative example (in a natural state), and FIG. 23 is a view (plan view) of the wire for removing an intravascular foreign body, which is shown in FIG. 22, when viewed from the distal end side thereof.

Hereinafter, with reference to the drawings, the of the wire for removing an intravascular foreign body will be described. It is noted that the description will be made of differences from the embodiments and comparative examples described above, and points which are the same as those associated with the previous embodiments and comparative examples will not be repeated.

This comparative example is similar to the fifth comparative example except that the positional relationship among the bent and deformed portions is different.

In the capturing portion 3 of a wire 1H for removing an intravascular foreign body shown in FIGS. 22 and 23, the respective crest portions (respective inflection points 16) of the bent and deformed portions 15f enter the respective trough (or valley) portions (respective inflection points 16) of the bent and deformed portion 15d. Further, the respective crest portions of the bent and deformed portion 15f enter the respective trough portions of the bent and deformed portion 15e.

In the capturing portion 3 constituted as described above, the void volumes of the gaps 32 and 33 are set even smaller. Therefore, the embolus 200 housed in the capturing portion 3 can be more surely prevented from leaving the gaps 32 and 33. Thus, the embolus 200 can be captured and removed more surely.

Note that the capturing portion 3 is constituted such that the filaments adjacent to each other are spaced apart from each other in the illustration though the crest portions of one of the filaments enter the trough portions of the other. However, the construction is not limited to this, the adjacent bent and deformed portions may partially contact or intersect each other. In this case, the void volumes in the capturing portion 3 are set further smaller, and therefore, the embolus 200 housed in the capturing portion 3 can be more surely prevented from leaving the gaps 32 and 33.

Seventh comparative example

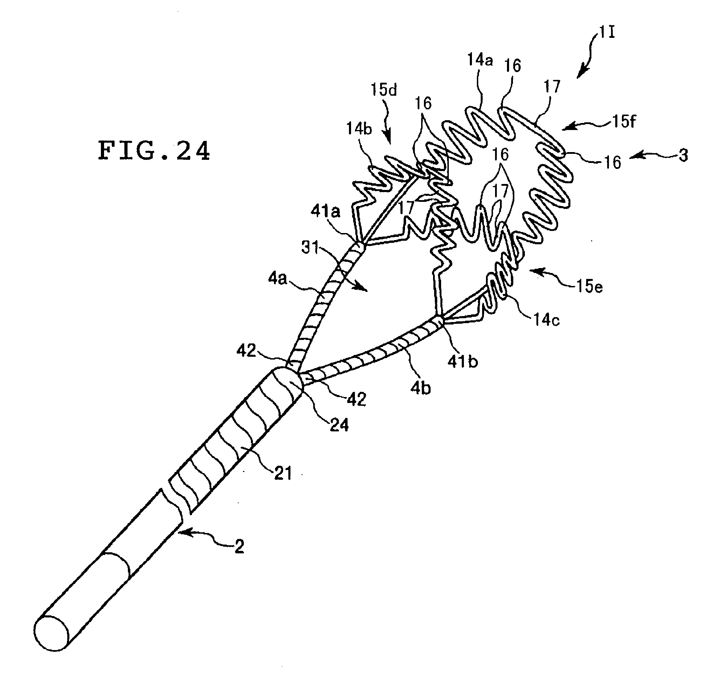

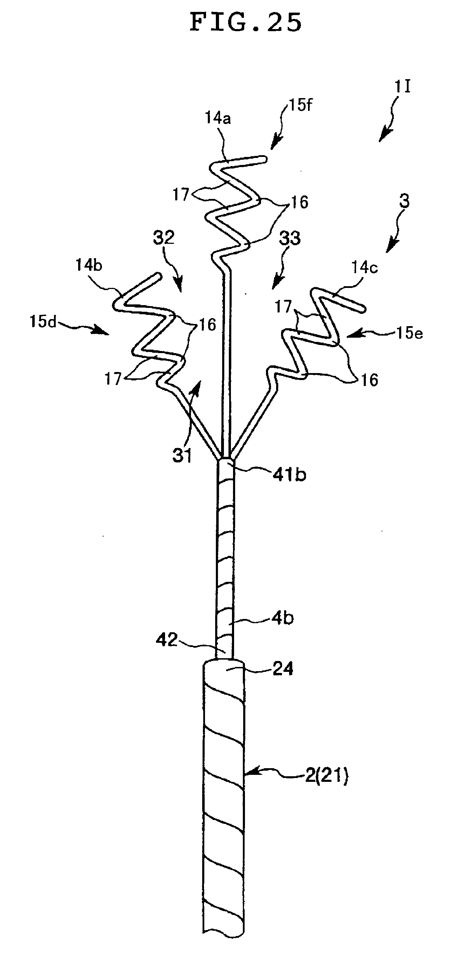

FIG. 24 is a perspective view showing a wire for removing an intravascular foreign body according to a seventh comparative example (in a natural state), and FIG. 25 is a side view of the wire for removing an intravascular foreign body, which is shown in FIG. 24.

A description will be made below of the seventh comparative example of the wire for removing an intravascular foreign body with reference to FIGS. 24 and 25. However, the description will be made mainly of differences from those of the above-described embodiments and comparative examples, and a description of similar matters will be omitted.

This comparative example is similar to thesixth comparative example except that the positional relationship among the bent and deformed portions is different.

In the capturing portion 3 of a wire 1I for removing an intravascular foreign object, which is shown in FIG. 24 and FIG. 25, the bent and deformed portion 15f is located distal to (on the distal end side than) the bent and deformed portions 15d and 15e. Thus, in the contracted state of the capturing portion 3, the bent and deformed portion 15f is located distal to (on the distal end side than) the bent and deformed portions 15d and 15e, and the bent and deformed portions 15d, 15e and 15f can be prevented from being intertwined with each other.

Eigth comparative example

A description will be made below of a wire for removing an intravascular foreign body according to an eigth comparative example of the present invention. However, the description will be made mainly of differences from those of the above-described embodiments, and a description of similar matters will be omitted.

This comparative example is similar to the seventh comparative example except that the positional relationship among the bent and deformed portions is different.

Note that in the capturing portion of the wire for removing an intravascular foreign object according to the eleventh embodiment, the bent and deformed portion 15f is proximal to (on the proximal end side than) the bent and deformed portions 15d and 15e. Further, in the contracted state of the capturing portion 3, the bent and deformed portion 15f is located proximal to (on the proximal end side than) the bent and deformed portions 15d and 15e. Also with this configuration, the bent and deformed portions 15d, 15e and 15f can be prevented from being intertwined with each other.

However, the bent and deformed portion 15f is located distal to the bent and deformed portions 15d and 15e, and thus, when the size of the center filament 14a is set to be the same as that in the case where the bent and deformed portion 15a is located proximal to (on the proximal end side than) the portion 15d and 15e, the lengths and widths of the filaments 14b and 14c in the longitudinal direction can be suppressed as compared with the above-described case. Accordingly, the filaments 14b and 14c on both sides can be set small. Therefore, the capturing portion 3 can be reduced in size while ensuring the size of the foreign object capturing space 31.

The description has been made above of the wire for removing an intravascular foreign body and the medical instrument according to the present invention based on the illustrated embodiments and comparative examples.Further, arbitrary components may be added.

Further, each of the wire for removing an intravascular foreign body and the medical instrument according to the present invention may be one formed by combining two or more arbitrary constructions (features) in the above-described embodiments.

For example, the third filaments of the third embodiment may be provided on the second filaments of the first embodiment.

The number of branch wires is not limited to two; and may be three or more.

Further, the number of formed first filaments (installation number thereof)is not limited to three; and may be two, four, or more.

Further, the number of formed second filaments is not limited to two and may be, for example, three or more.

Further, the construction is not limited to the construction in which the third filaments are respectively provided on the plurality of second filaments, and for example, the third filaments may be provided on one second filament.

Further, the number of formed third filaments is not limited to two and may be, for example, one or three or more.

Further, when the plurality of third filaments are provided, the construction is not limited to the construction in which the positions of the branch points thereof are different from each other, and the positions may substantially coincide with each other.

A wire for removing an intravascular foreign body according to the present invention is a wire for removing an intravascular foreign body, including: a long or elongated wire body with flexibility; and a capturing portion including a foreign body capturing space which captures a foreign body in a blood vessel therein, the capturing portion being provided on a distal end of the wire body, in which the capturing portion includes: at least two branch wires branching from the distal end of the wire body; and a plurality of filaments bridged between the two branch wires, and at least one of the filaments includes a plurality of curved projections bent in a direction deviated from an extension line of a central axis of a proximal end of the filament. In accordance with the wire for removing an intravascular foreign body according to the present invention, the foreign body in the blood vessel can be surely captured and removed.

The scope of the invention is merely defined by the claims.

**Claims**

1. A wire (1) for removing an intravascular foreign body, comprising:

a long or elongated wire body (2) with flexibility; and
a capturing portion (3) including a foreign body capturing space (31) for capturing a foreign body (200) in a blood vessel (100) in the foreign body capturing space, the capturing portion being provided on a distal end of the wire body, wherein the capturing portion includes:

at least two branch wires (4a, 4b) branching from the distal end of the wire body; and
a plurality of first filaments (5a, 5b, 5c) bridged between the two branch wires, **characterized in that**

**characterized in that**
at least one of the first filaments (5a, 5b, 5c) includes a plurality of curved projections (7a, 7b, 7c, 7d) being second filaments (7a, 7b, 7c, 7d) bent in a direction deviating from a plane (52b, 52c), defined by the first filaments (5a, 5b, 5c) in a natural state, each of said second filaments (7a, 7b, 7c, 7d) forming a loop having each of both ends at two different points on the at least one of the first filaments (5a, 5b, 5c).

2. The wire for removing an intravascular foreign body according to claim 1, wherein each of the plurality of curved projections (7a, 7b, 7c, 7d) includes a first curved projection (7a, 7c) bent in a direction deviated in a first direction from the plane, defined by the first filament (5b, 5c) in a natural state, and a second curved projection (7b, 7d) bent in a direction deviated from the plane, defined by the first filament (5b, 5c) opposite to the first direction.

3. A wire for removing an intravascular foreign body according to claim 1,
wherein the second filaments (7a, 7b, 7c, 7d) are branching from midway of the at least one of the first filaments (5a, 5b, 5c).

4. The wire for removing an intravascular foreign body according to claim 3, wherein a sum of $\alpha$ and $\beta$ satisfies a following expression in a natural state:

$$45° \leq \alpha + \beta \leq 90°$$

where $\alpha$ is an angle made by a plane defined by the branch wires (4a, 4b) and the plane defined by the first filaments (5a, 5b, 5c) found in a plane different than the plane of the branch wires, and $\beta$ is an angle made by the plane defined by the first filaments (5a, 5b, 5c) and a plane defined by each of the second filaments (7a, 7b, 7c, 7d) found on the respective first filament.

5. The wire for removing an intravascular foreign body according to claim 3, wherein positions of branch points (54a, 54b, 54c, 54d) of the plurality of second filaments (7a, 7b, 7c, 7d) coincide with each other substantially.

6. The wire for removing an intravascular foreign body according to claim 3, wherein positions of branch points (54e, 54f, 54g, 54h) of the plurality of second filaments are different to each other.

7. The wire for removing an intravascular foreign body according to any one of claims 3 to 6, wherein the capturing portion (3) includes a third filament (13a, 13b, 13c, 13d, 13e, 13f) on at least one of the second filaments (7e, 7f, 7g, 7h), the third filament (13a, 13b, 13c, 13d, 13e, 13f) branching from midway of the at least one of the second filament (7e, 7f, 7g, 7h).

8. The wire for removing an intravascular foreign body according to claim 7, wherein a plurality of the third filaments (13a, 13b, 13c, 13d, 13e, 13f) are provided on the at least one of the second filaments (7e, 7f, 7g, 7h).

9. The wire for removing an intravascular foreign body according to claim 3,
wherein at least one of the filaments (14a, 14b, 14c) includes a bent and deformed portion (15a) having a plurality of bent or steeply curved inflection points (16), and a coupling portion (17) located between two of the inflection points (16) and disposed to enter a space between the filaments adjacent to each other.

10. The wire for removing an intravascular foreign body according to claim 9, wherein the bent and deformed portion (15a) has a plurality of the coupling portions (17), and the coupling portions (17) adjacent to each other are extended in directions different from each other.

11. The wire for removing an intravascular foreign body according to claim 10, wherein the bent and deformed portion (15d) forms a wave shape when viewed from a distal end side of the wire body in a longitudinal direction.

12. The wire for removing an intravascular foreign body according to any one of claims 10 and 11, wherein each of the plurality of filaments (14a, 14b, 14c) has the bent and deformed portion (15d) forming a wave shape, and in the bent and deformed portion (15a) adjacent to each other, each of the inflection points (16) of one of the filaments (14a, 14b, 14c) enters a space between the two coupling portions of the other.

13. The wire for removing an intravascular foreign body according to any one of claims 11 and 12, wherein, in the bent and deformed portion (15d) forming the wave shape, amplitude of the bent and deformed portion on the distal end side is larger than amplitude of the bent and deformed portion on a proximal end side.

14. The wire for removing an intravascular foreign body according to any one of claims 9 to 13, wherein the bent and deformed portion is composed of a linear body having portions different in thickness.

15. The wire for removing an intravascular foreign body according to any one of claims 9 to 14, wherein each of the plurality of filaments has the bent and deformed portion, and the bent and deformed portion partially contact or intersect each other.

16. The wire for removing an intravascular foreign body according to any one of claims 9 to 15, wherein the bent and deformed portion is superior in pliability than the branch wires.

17. The wire for removing an intravascular foreign body according to any one of claims 9 to 16, wherein the plurality of filaments are capable of approaching and being spaced apart from one another.

18. A medical instrument, comprising:

the wire for removing an intravascular foreign body according to any one of claims 1 to 17; and a catheter (2, 21) provided with a lumen capable of housing the wire for removing an intravascular foreign body in the lumen.

19. The medical instrument according to claim 18, wherein distal ends of the branch wires (4a, 4b) are suitable to be spaced apart from each other when the capturing portion is allowed to project from a distal end opening of the lumen; and the distal ends of the branch wires (4a, 4b) are suitable to come close to each other by being regulated by an inner wall surface which defines the lumen when the capturing portion is housed in the lumen.

**Patentansprüche**

1. Draht (1) zum Entfernen eines intravaskulären Fremdkörpers mit:

einem langen oder gestreckten Drahtkörper (2), der Flexibilität aufweist; und einem Einfangabschnitt (3) einschließlich eines Fremdkörpereinfangraums (31) zum Einfangen eines Fremdkörpers (200) in einem Blutgefäß (100) in dem Fremdkörpereinfangraum, wobei der Einfangabschnitt an einem distalen Ende des Drahtkörpers vorgesehen ist, wobei der Einfangabschnitt Folgendes aufweist:

wenigstens zwei Zweigdrähte (4a, 4b), die von dem distalen Ende des Drahtkörpers abzweigen, und eine Vielzahl von ersten Filamenten (5a, 5b, 5c), die zwischen den zwei Zweigdrähten überbrücken,

**gekennzeichnet dadurch, dass**
wenigstens eines von den ersten Filamenten (5a, 5b, 5c) eine Vielzahl von gekrümmten Vorsprüngen (7a, 7b, 7c, 7d) als zweite Filamente (7a, 7b, 7c, 7d) aufweist, die in eine Richtung gebogen sind, welche von einer durch die ersten Filamente (5a, 5b, 5c) in einem ursprünglichen Zustand festgelegten Ebene (52b, 52c) abweicht, wobei jedes von den zweiten Filamenten (7a, 7b, 7c, 7d) eine Schleife ausbildet, deren beide Enden an zwei verschiedenen Punkten an dem wenigsten einen von den ersten Filamenten (5a, 5b, 5c) angeordnet sind.

2. Draht zum Entfernen eines intravaskulären Fremdkörpers nach Anspruch 1, wobei jeder von der Vielzahl von gekrümmten Vorsprüngen (7a, 7b, 7c, 7d) einen ersten gekrümmten Vorsprung (7a, 7c), der in eine Richtung gebogen ist, die von der durch das erste Filament (5b, 5c) in einem natürlichen Zustand festgelegten Ebene in eine erste Richtung abweicht, und einen zweiten gekrümmten Vorsprung (7b, 7d) aufweist, der in eine Richtung gebogen ist, die von der durch das erste Filament (5b, 5c) gegensätzlich zu der ersten Richtung festgelegten Ebene abweicht.

3. Draht zum Entfernen eines intravaskulären Fremdkörpers nach Anspruch 1,
wobei die zweiten Filamente (7a, 7b, 7c, 7d) auf halbem Wege von dem wenigstens einen von den ersten Filamenten (5a, 5b, 5c) abzweigen.

4. Draht zum Entfernen eines intravaskulären Fremdkörpers nach Anspruch 3, wobei eine Summe von α und β einen nachfolgenden Ausdruck in einem natürlichen Zustand erfüllt:

$$45° \leq \alpha + \beta \leq 90°,$$

wobei α ein Winkel ist, der von einer durch die Zweigdrähte (4a, 4b) festgelegten Ebene und der Ebene eingenommen wird, die durch die ersten Filamente (5a, 5b, 5c) festgelegt ist, die in einer von der Ebene der Zweigdrähte verschiedenen Ebene vorgefunden werden, und wobei β ein Winkel ist, der von der durch die ersten Filamente (5a, 5b, 5c) festgelegten Ebene und einer Ebene eingenommen wird, die durch jedes von den zweiten Filamenten (7a, 7b, 7c, 7d) festgelegt ist, die an dem entsprechenden ersten Filament vorgefunden werden.

5. Draht zum Entfernen eines intravaskulären Fremdkörpers nach Anspruch 3, wobei Positionen von Abzweigpunkten (54a, 54b, 54c, 54d) der Vielzahl von zweiten Filamenten (7a, 7b, 7c, 7d) im Wesentlichen miteinander zusammenfallen.

6. Draht zum Entfernen eines intravaskulären Fremdkörpers nach Anspruch 3, wobei Positionen von Abzweigpunkten (54e, 54f, 54g, 54h) der Vielzahl von zweiten Filamenten voneinander verschieden sind.

7. Draht zum Entfernen eines intravaskulären Fremdkörpers nach einem der Ansprüche 3 bis 6, wobei der Einfangabschnitt (3) ein drittes Filament (13a, 13b, 13c, 13d, 13e, 13f) an dem wenigstens einen von den zweiten Filamenten (7e, 7f, 7g, 7h) aufweist, wobei das dritte Filament (13a, 13b, 13c, 13d, 13e, 13f) auf halbem Wege von dem wenigstens einen von den zweiten Filamenten (7e, 7f, 7g, 7h) abzweigt.

8. Draht zum Entfernen eines intravaskulären Fremdkörpers nach Anspruch 7, wobei eine Vielzahl der dritten Filamente (13a, 13b, 13c, 13d, 13e, 13f) an dem wenigstens einen von den zweiten Filamenten (7e, 7f, 7g, 7h) vorgesehen sind.

9. Draht zum Entfernen eines intravaskulären Fremdkörpers nach Anspruch 3,
wobei wenigstens eines von den Filamenten (14a, 14b, 14c) einen gebogenen und verformten Abschnitt (15a) mit einer Vielzahl von gebogenen und steil gekrümmten Biegungspunkten (16) und einen Verbindungsabschnitt (17) aufweist, der sich zwischen zwei der Biegungspunkte (16) befindet und angeordnet ist, um in einen Raum zwischen den zueinander benachbarten Filamenten einzudringen.

10. Draht zum Entfernen eines intravaskulären Fremdkörpers nach Anspruch 9, wobei der gebogene und verformte Abschnitt (15a) eine Vielzahl der Verbindungsabschnitte (17) aufweist, und wobei sich die zueinander benachbarten Verbindungsabschnitte (17) in voneinander verschiedene Richtungen erstrecken.

11. Draht zum Entfernen eines intravaskulären Fremdkörpers nach Anspruch 10, wobei der gebogene und verformte Abschnitt (15d) aus Sicht einer distalen Endseite des Drahtkörpers in einer Längsrichtung eine Wellengestalt ausbildet.

**12.** Draht zum Entfernen eines intravaskulären Fremdkörpers nach einem der Ansprüche 10 und 11, wobei jedes von der Vielzahl von Filamenten (14, 14b, 14c) den gebogenen und verformten Abschnitt (15d) aufweist, der eine Wellengestalt ausbildet, und wobei in den zueinander benachbarten gebogenen und verformten Abschnitten (15a) jeder der Biegungspunkte (16) von einem von den Filamenten (14a, 14b, 14c) in einen Raum zwischen den zwei Verbindungsabschnitten des anderen eindringt.

**13.** Draht zum Entfernen eines intravaskulären Fremdkörpers nach einem der Ansprüche 11 und 12, wobei bei dem gebogenen und verformten Abschnitt (15d), der die Wellengestalt ausbildet, eine Amplitude des gebogenen und verformten Abschnitts auf der distalen Endseite größer als eine Amplitude des gebogenen und verformten Abschnitts auf einer proximalen Endseite ist.

**14.** Draht zum Entfernen eines intravaskulären Fremdkörpers nach einem der Ansprüche 9 bis 13, wobei der gebogene und verformte Abschnitt aus einem linearen Körper besteht, der Abschnitte mit verschiedenen Dicken aufweist.

**15.** Draht zum Entfernen eines intravaskulären Fremdkörpers nach einem der Ansprüche 9 bis 14, wobei jedes von der Vielzahl von Filamenten den gebogenen und verformten Abschnitt aufweist, und wobei der gebogene und verformte Abschnitt teilweise den anderen berührt oder kreuzt.

**16.** Draht zum Entfernen eines intravaskulären Fremdkörpers nach einem der Ansprüche 9 bis 15, wobei der gebogene und verformte Abschnitt bezüglich der Biegsamkeit überlegener als die Zweigdrähte ist.

**17.** Draht zum Entfernen eines intravaskulären Fremdkörpers nach einem der Ansprüche 9 bis 16, wobei die Vielzahl von Filamenten in der Lage sind, einander zu erreichen und voneinander beabstandet zu sein.

**18.** Medizinisches Instrument mit:

dem Draht zum Entfernen eines intravaskulären Fremdkörpers nach einem der Ansprüche 1 bis 17; und
einem Katheter (2, 21), der mit einem Lumen versehen ist, das in der Lage ist, den Draht zum Entfernen eines intravaskulären Fremdkörpers in dem Lumen unterzubringen.

**19.** Medizinisches Instrument nach Anspruch 18, wobei distale Enden der Zweigdrähte (4a, 4b) angepasst sind, um voneinander beabstandet zu sein, wenn der Einfangabschnitt in der Lage ist, von einer distalen Endöffnung des Lumens vorzustehen; und die distalen Enden der Zweigdrähte (4a, 4b) angepasst sind, um durch Regulierung anhand einer Innenwandfläche einander näher zu kommen, die das Lumen festlegt, wenn der Einfangabschnitt in dem Lumen untergebracht ist.

## Revendications

**1.** Fil (1) pour enlever un corps étranger intravasculaire, comprenant :

un corps de fil (2) long ou allongé avec une flexibilité ; et
une partie de capture (3) incluant un espace de capture de corps étranger (31) pour capturer un corps étranger (200) dans un vaisseau sanguin (100) dans l'espace de capture de corps étranger, la partie de capture étant prévue sur une extrémité distale du corps de fil,
dans lequel la partie de capture inclut :

au moins deux fils ramifiés (4a, 4b) se ramifiant à partir de l'extrémité distale du corps de fil ; et
une pluralité de premiers filaments (5a, 5b, 5c) pontés entre les deux fils ramifiés,

**caractérisé en ce que**
au moins un des premiers filaments (5a, 5b, 5c) inclut une pluralité de projections incurvées (7a, 7b, 7c, 7d) étant des deuxièmes filaments (7a, 7b, 7c, 7d) pliés dans une direction déviée par rapport à un plan (52b, 52c), défini par les premiers filaments (5a, 5b, 5c) en un état naturel, chacun desdits deuxièmes filaments (7a, 7b, 7c, 7d) formant une boucle ayant chacune des deux extrémités en deux points différents sur l'au moins un des premiers filaments (5a, 5b, 5c).

**2.** Fil pour enlever un corps étranger intravasculaire selon la revendication 1, dans lequel chacune de la pluralité de projections incurvées (7a, 7b, 7c, 7d) inclut une première projection incurvée (7a, 7c) pliée dans une direction déviée dans une première direction par rapport au plan, défini par le premier filament (5b, 5c) en un état naturel, et une deuxième projection incurvée (7b, 7d) pliée dans une direction déviée par rapport au plan défini par le premier filament (5b, 5c) opposée à la première direction.

**3.** Fil pour enlever un corps étranger intravasculaire selon la revendication 1, dans lequel les deuxièmes filaments (7a, 7b, 7c, 7d) se ramifient à mi-chemin de l'au moins un des premiers filaments (5a, 5b, 5c).

4. Fil pour enlever un corps étranger intravasculaire selon la revendication 3, dans lequel une somme de α et β satisfait à une expression suivante en un état naturel :

$$45° \leq \alpha + \beta \leq 90°$$

où α est un angle constitué par un plan défini par les fils ramifiés (4a, 4b) et le plan défini par les premiers filaments (5a, 5b, 5c) se trouvant dans un plan différent du plan des fils ramifiés, et β est un angle constitué par le plan défini par les premiers filaments (5a, 5b, 5c) et un plan défini par chacun des deuxièmes filaments (7a, 7b, 7c, 7d) se trouvant sur le premier filament respectif.

5. Fil pour enlever un corps étranger intravasculaire selon la revendication 3, dans lequel des positions de points de ramification (54a, 54b, 54c, 54d) de la pluralité de deuxièmes filaments (7a, 7b, 7c, 7d) coïncident substantiellement les unes avec les autres.

6. Fil pour enlever un corps étranger intravasculaire selon la revendication 3, dans lequel des positions de points de ramification (54e, 54f, 54g, 54h) de la pluralité de deuxièmes filaments sont différentes les unes des autres.

7. Fil pour enlever un corps étranger intravasculaire selon l'une quelconque des revendications 3 à 6, dans lequel la partie de capture (3) inclut un troisième filament (13a, 13b, 13c, 13d, 13e, 13f) sur au moins un des deuxièmes filaments (7e, 7f, 7g, 7h), le troisième filament (13a, 13b, 13c, 13d, 13e, 13f) se ramifiant à mi-chemin de l'au moins un des deuxièmes filaments (7e, 7f, 7g, 7h).

8. Fil pour enlever un corps étranger intravasculaire selon la revendication 7, dans lequel une pluralité des troisièmes filaments (13a, 13b, 13c, 13d, 13e, 13f) sont prévus sur l'au moins un des deuxièmes filaments (7e, 7f, 7g, 7h).

9. Fil pour enlever un corps étranger intravasculaire selon la revendication 3,
dans lequel au moins un des filaments (14a, 14b, 14c) inclut une partie pliée et déformée (15a) ayant une pluralité de points d'inflexion (16) pliés ou fortement incurvés, et une partie de couplage (17) située entre deux des points d'inflexion (16) et disposée de façon à pénétrer dans un espace entre les filaments adjacents les uns aux autres.

10. Fil pour enlever un corps étranger intravasculaire selon la revendication 9, dans lequel la partie pliée et déformée (15a) a une pluralité des parties de couplage (17), et les parties de couplage (17) adjacentes les unes aux autres sont étendues dans des directions différentes les unes des autres.

11. Fil pour enlever un corps étranger intravasculaire selon la revendication 10, dans lequel la partie pliée et déformée (15d) forme une forme d'onde lorsqu'elle est vue d'un côté d'extrémité distale du corps de fil dans un sens longitudinal.

12. Fil pour enlever un corps étranger intravasculaire selon l'une quelconque des revendications 10 et 11, dans lequel chacun de la pluralité de filaments (14a, 14b, 14c) a la partie pliée et déformée (15d) formant une forme d'onde, et dans la partie pliée et déformée (15a) adjacente l'une à l'autre, chacun des points d'inflexion (16) de l'un des filaments (14a, 14b, 14c) pénètre dans un espace entre les deux parties de couplage de l'autre.

13. Fil pour enlever un corps étranger intravasculaire selon l'une quelconque des revendications 11 et 12, dans lequel, dans la partie pliée et déformée (15d) formant la forme d'onde, une amplitude de la partie pliée et déformée sur le côté d'extrémité distale est plus grande qu'une amplitude de la partie pliée et déformée sur un côté d'extrémité proximale.

14. Fil pour enlever un corps étranger intravasculaire selon l'une quelconque des revendications 9 à 13, dans lequel la partie pliée et déformée est composée d'un corps linéaire ayant des parties différentes en épaisseur.

15. Fil pour enlever un corps étranger intravasculaire selon l'une quelconque des revendications 9 à 14, dans lequel chacun de la pluralité de filaments a la partie pliée et déformée, et la partie pliée et déformée est en contact partiel ou en intersection l'une avec l'autre.

16. Fil pour enlever un corps étranger intravasculaire selon l'une quelconque des revendications 9 à 15, dans lequel la partie pliée et déformée est supérieure en pliabilité aux fils ramifiés.

17. Fil pour enlever un corps étranger intravasculaire selon l'une quelconque des revendications 9 à 16, dans lequel la pluralité de filaments sont aptes à s'approcher et à être espacés les uns des autres.

18. Instrument médical, comprenant :

le fil pour enlever un corps étranger intravasculaire selon l'une quelconque des revendications 1 à 17 ; et

un cathéter (2, 21) prévu avec une lumière apte à recevoir le fil pour enlever un corps étranger intravasculaire dans la lumière.

**19.** Instrument médical selon la revendication 18, dans lequel des extrémités distales des fils ramifiés (4a, 4b) sont appropriées à être espacées l'une de l'autre lorsque la partie de capture est laissée se projeter d'une ouverture d'extrémité distale de la lumière ; et

les extrémités distales des fils ramifiés (4a, 4b) sont appropriées à se rapprocher l'une de l'autre en étant régulées par une surface de paroi intérieure qui définit la lumière lorsque la partie de capture est reçue dans la lumière.

EP 1 736 106 B1

FIG. 1

24

# FIG.2

# FIG.3

# FIG.4

# FIG.5

EP 1 736 106 B1

# FIG.6

EP 1 736 106 B1

FIG.7

EP 1 736 106 B1

# FIG.8

FIG.9

EP 1 736 106 B1

# FIG.10

FIG.11

EP 1 736 106 B1

# FIG.12

# FIG.13

EP 1 736 106 B1

FIG.14

EP 1 736 106 B1

# FIG.15

# FIG.16

FIG.17

EP 1 736 106 B1

# FIG.18

EP 1 736 106 B1

# FIG.19

41

FIG.20A

FIG.20B

FIG.20C

# FIG.21

# FIG.22

# FIG.23

EP 1 736 106 B1

FIG.24

46

# FIG.25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004016668 A

- JP 62268545 A